(19) Europäisches Patentamt
European Patent Office
Office européen des brevets

(11) EP 1 746 169 B1

(12) EUROPÄISCHE PATENTSCHRIFT

(45) Veröffentlichungstag und Bekanntmachung des Hinweises auf die Patenterteilung:
**23.12.2009 Patentblatt 2009/52**

(51) Int Cl.:
***C12Q 1/68*** *(2006.01)* ***G06F 19/00*** *(2006.01)*

(21) Anmeldenummer: **05090216.2**

(22) Anmeldetag: **21.07.2005**

(54) **Verfahren zur Quantifizierung methylierter DNA**

Method for quantification of methylated DNA

Procédé de quantification d'ADN méthylé

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**24.01.2007 Patentblatt 2007/04**

(73) Patentinhaber: **Epigenomics AG**
**10178 Berlin (DE)**

(72) Erfinder: **Tetzner, Reimo**
**10439 Berlin (DE)**

(74) Vertreter: **Schubert, Klemens**
**Neue Promenade 5**
**10178 Berlin-Mitte (DE)**

(56) Entgegenhaltungen:
**WO-A-03/081532 US-B1- 6 331 393**

- **TRINH BINH N ET AL: "DNA methylation analysis by MethyLight technology" METHODS : A COMPANION TO METHODS IN ENZYMOLOGY, ACADEMIC PRESS INC., NEW YORK, NY, US, Bd. 25, Nr. 4, Dezember 2001 (2001-12), Seiten 456-462, XP002318911 ISSN: 1046-2023**
- **RAND KEITH ET AL: "Conversion-specific detection of DNA methylation using real-time polymerase chain reaction (ConLight-MSP) to avoid false positives" METHODS (ORLANDO), Bd. 27, Nr. 2, Juni 2002 (2002-06), Seiten 114-120, XP002296074 ISSN: 1046-2023**
- **OLIVER D H ET AL: "Use of single nucleotide polymorphisms (SNP) and real-time polymerase chain reaction for bone marrow engraftment analysis." THE JOURNAL OF MOLECULAR DIAGNOSTICS : JMD. NOV 2000, Bd. 2, Nr. 4, November 2000 (2000-11), Seiten 202-208, XP002296075 ISSN: 1525-1578**
- **LIVAK K J: "ALLELIC DISCRIMINATION USING FLUOROGENIC PROBES AND THE 5' NUCLEASEASSAY" GENETIC ANALYSIS: BIOMOLECULAR ENGINEERING, ELSEVIER SCIENCE PUBLISHING, US, Bd. 14, 1999, Seiten 143-149, XP002944060 ISSN: 1050-3862**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 746 169 B1

Sequence listing

<110> Epigenomics AG

<120> Verfahren zur Quantifizierung methylierter DNA

<160> 13

<210> 1
<211> 24
<212> DNA
<213> Artificial Sequence

<220>
<223> chemically treated genomic DNA (Homo sapiens)

<400> 1
ttattgatgg ttgttggata tttt 24

<210> 2
<211> 20
<212> DNA
<213> Artificial Sequence

<220>
<223> chemically treated genomic DNA (Homo sapiens)

<400> 2
aaaacctacc taccctccta 20

<210> 3
<211> 29
<212> DNA
<213> Artificial Sequence

<220>
<223> chemically treated genomic DNA (Homo sapiens)

<400> 3
tggttttttt gaatttttg agtttttg 29

<210> 4
<211> 19
<212> DNA
<213> Artificial Sequence

<220>
<223> chemically treated genomic DNA (Homo sapiens)

<400> 4
cgattagggg acgatgacg 19

<210> 5
<211> 161
<212> DNA
<213> Artificial Sequence

<220>
<223> chemically treated genomic DNA (Homo sapiens)

<400> 5

ttattgatgg ttgttggata ttttcgaaga ggtttttcg tgggcggggt ttttgggtgg 60
taatacgatt aggggacgat gacgtttggt ttttttgaat tttttgagtt ttttggtaag 120
tatgcgattt cggcgggtac gtaggagggt aggtaggttt t 161

<210> 6
<211> 24
<212> DNA
<213> Artificial Sequence

<220>
<223> chemically treated genomic DNA (Homo sapiens)

<400> 6
ttattgatgg ttgttggata tttt 24

<210> 7
<211> 20
<212> DNA
<213> Artificial Sequence

<220>
<223> chemically treated genomic DNA (Homo sapiens)

<400> 7
aaaacctacc taccctccta 20

<210> 8
<211> 29
<212> DNA
<213> Artificial Sequence

<220>
<223> chemically treated genomic DNA (Homo sapiens)

<400> 8
tggttttttt gaatttttg agtttttg 29

<210> 9
<211> 29
<212> DNA
<213> Artificial Sequence

<220>
<223> chemically treated genomic DNA (Homo sapiens)

<400> 9
tggttttatt gaatgttttg agttttttg 29

<210> 10
<211> 20
<212> DNA
<213> Artificial Sequence

<220>
<223> chemically treated genomic DNA (Homo sapiens)

<400> 10
taagtatgcg atttcggcgg 20

<210> 11
<211> 24
<212> DNA
<213> Artificial Sequence

<220>
<223> chemically treated genomic DNA (Homo sapiens)

<400> 11
taagtatgtg attttggtgg gtat 24

<210> 12
<211> 161
<212> DNA
<213> Artificial Sequence

<220>
<223> chemically treated genomic DNA (Homo sapiens)

<400> 12

```
ttattgatgg ttgttggata ttttcgaaga ggtttttttcg tgggcggggt ttttgggtgg      60
taatacgatt aggggacgat gacgtttggt ttttttgaat tttttgagtt ttttggtaag     120
tatgcgattt cggcgggtac gtaggagggt aggtaggttt t                         161
```

<210> 13
<211> 161
<212> DNA
<213> Artificial Sequence

<220>
<223> chemically treated genomic DNA (Homo sapiens)

<400> 13

```
ttattgatgg ttgttggata ttttcgaaga ggttttttcg tgggcggggt ttttgggtgg      60
taatacgatt aggggacgat gacgtttggt tttattgaat gttttgagtt ttttggtaag     120
tatgcgattt cggcgggtac gtaggagggt aggtaggttt t                         161
```

## Beschreibung

Die vorliegende Erfindung betrifft ein Verfahren zur Quantifizierung von methylierten Cytosinpositionen in DNA. 5-Methylcytosin ist die häufigste kovalent modifizierte Base in der DNA eukaryotischer Zellen. Sie spielt eine wichtige biologische Rolle, u.a. bei der Transkriptionsregulation, beim genetischen Imprinting und in der Tumorgenese (zur Übersicht: Millar et al.: Five not four: History and significance of the fifth base. In: The Epigenome, S. Beck and A. Olek (eds.), Wiley-VCH Verlag Weinheim 2003, S. 3-20). Die Identifizierung von 5-Methylcytosin ist insbesondere für die Krebsdiagnostik von erheblichem Interesse. Ein Nachweis von Methylcytosin ist allerdings schwierig, da Cytosin und Methylcytosin das gleiche Basenpaarungsverhalten aufweisen. Die herkömmlichen, auf Hybridisierung beruhenden DNA-Analyseverfahren sind daher nicht anwendbar. Dementsprechend arbeiten die gängigen Methoden zur Methylierungsanalyse nach zwei unterschiedlichen Prinzipien. Zum einen werden methylierungsspezifische Restriktionsenzyme benutzt, zum anderen erfolgt eine selektive chemische Umwandlung von nicht-methylierten Cytosinen in Uracil (sog.: Bisulfit-Behandlung, siehe etwa: DE 101 54 317 A1; DE 100 29 915 A1). Die enzymatisch oder chemisch vorbehandelte DNA wird dann meist amplifiziert und kann auf unterschiedliche Weise analysiert werden (zur Übersicht: WO 02/072880 S. 1 ff; Fraga and Estella: DNA methylation: a profile of methods and applications. Biotechniques. 2002 Sep; 33(3): 632, 634, 636-49.). Zur sensitiven Analyse wird die chemisch vorbehandelte DNA üblicherweise mittels eines PCR-Verfahrens amplifiziert. Eine selektive Amplifikation nur der methylierten (bzw. bei umgekehrten Ansatz: unmethylierten) DNA kann über die Verwendung methylierungsspezifischer Primer oder Blocker gewährleistet werden (sog. methylierungssensitive PCR/MSP bzw. "Heavy Methyl-Verfahren" , vgl.: Herman et al.: Methylation-specific PCR: a novel PCR assay for methylation status of CpG islands. Proc Natl Acad Sei U S A. 1996 Sep 3; 93(18):9821-6 Cottrell et al.: A real-time PCR assay for DNAmethylation using methylation-specific blockers. Nucl. Acids. Res. 2004 32: e10). Auf der anderen Seite ist es auch möglich, die DNA zunächst methylierungsunspezifisch zu amplifizieren und dann die Amplifikate mittels methylierungsspezifischer Sonden zu analysieren (zur Übersicht: Trinh et al.: DNA methylation analysis by MethyLight technology. Methods. 2001 Dec;25(4):456-62). Die genannten PCR-Verfahren sind auch als Real-Time-PCR-Varianten anwendbar. Diese ermöglichen es, den Methylierungsstatus direkt im Verlauf der PCR nachzuweisen, ohne dass eine nachfolgende Analyse der Produkte erforderlich wäre ("MethyLight" - WO00/70090; US 6,331,393; Trinh et al. 2001, a.a.o.).

Eine Quantifizierung des Methylierungsgrades ist für verschiedene Anwendungen erforderlich, etwa für Klassifizierungen von Tumoren, für prognostische Aussagen oder für die Vorhersage von Arzneimittelwirkungen. Es sind unterschiedliche Verfahren zur Quantifizierung des Methylierungsgrades bekannt. Zum Teil erfolgt dabei zunächst eine Amplifikation der DNA, etwa bei Ms-SNuPE, bei Hybridisierungen auf Microarrays, bei Hybridisierungsassays in Lösung oder bei der direkten Bisulfit-Sequenzierung (zur Übersicht: Fraga and Estella 2002, a.a.o.). Ein Problem bei diesen "Endpunktanalysen" besteht darin, dass die Amplifikation u.a. aufgrund Produkthemmung, Enzyminstabilität und Konzentrationsabnahme der Reaktionskomponenten ungleichmäßig erfolgen kann. Eine Korrelation zwischen der Menge an Amplifikat und der Menge an eingesetzter DNA ist daher nicht immer gegeben. Die Quantifizierung wird daher fehleranfällig (vgl.: Kains: The PCR plateau phase - towards an understanding of its limitations. Biochem. Biophys. Acta 1494 (2000) 23-27). Die auf einer Real-Time-PCR basierende Schwellenwertanalyse bestimmt die Menge an Amplifikat dagegen nicht am Ende der Amplifikation, sondern in der exponentiellen Amplifikationsphase. Diese Methode setzt voraus, dass die Amplifikationseffizienz in der exponentiellen Phase konstant ist. Der sog. Schwellenwert Ct ist ein Maß für denjenigen PCR-Zyklus, bei dem das Signal in der exponentiellen Phase der Amplifikation zum ersten Mal größer als das Hintergrundrauschen ist. Die absolute Quantifizierung erfolgt dann über einen Vergleich des Ct-Werts der untersuchten DNA mit dem Ct-Wert eines Standards (vgl.: Trinh et al. 2001, a.a.o.; Lehmann et al.: Quantitative assessment of promoter hypermethylation during breast cancer development. Am J Pathol. 2002 Feb;160(2):605-12). Ein Problem der Ct-Analyse besteht allerdings darin, dass bei hohen DNA-Konzentrationen nur eine geringe Auflösung erreicht werden kann. Das gleich gilt, wenn hohe Methylierungsgrade über PMR-Werte ermittelt werden sollen (vgl. zu PMR-Werten: Eads et al., CANCER RESEARCH 61, 3410-3418, April 15, 2001.) Zudem ist für diese Art der Ct-Analyse auch die Amplifkation eines Referenzgens, etwa des β-Aktin-Gens, erforderlich (vgl.: Trinh et al 2001, a.a.o.).

Kürzlich wurde ein Verfahren zur quantitativen Methylierungsanalyse beschrieben, bei dem die bisulfitumgewandelte DNA amplifiziert und mittels zweier methylierungsspezifischer Real-Time-Sonden detektiert wird ("QM"-Assay). Dabei ist die eine Sonde spezifisch für den methylierten Zustand, während die andere Sonde spezifisch für den unmethylierten Zustand ist. Die beiden Sonden tragen unterschiedliche Fluoreszenzfarbstoffe. Innerhalb bestimmter PCR-Zyklen kann dann etwa über das Verhältnis der Signalintensitäten der beiden Sonden oder über die Cts der Fluoreszenzkanäle eine Quantifizierung des Methylierungsgrades erfolgen (vgl.: Lehmann and Kreipe: Real-time PCR-based assay for quantitative determination of methylationstatus. Methods Mol Biol 2004; 287:207-18; Zeschnigk et al.: A novel real-time PCR assay for quantitative analysis of methylated alleles (QAMA): analysis of the retinoblastoma locus. Nucleic Acids Res. 2004 Sep 07; 32(16):e125.)

Im Folgenden ist ein weiteres Real-Time-PCR Verfahren zur Quantifizierung methylierter DNA beschrieben. Dabei wird ebenfalls bisulfitierte DNA amplifiziert und mittels zweier Real-Time-Sonden detektiert. Anders als bei dem oben beschriebenen Verfahren ist jedoch nur eine der Sonden spezifisch für den methylierten (bzw. den unmethylierten) Zustand. Die andere Sonde ist methylierungsunspezifisch. Mit Hilfe dieser beiden Sonden ist es erfindungsgemäß möglich, eine einfache Quantifizierung der Cytosinmethylierung durchzuführen.

Ein prinzipieller Unterschied des erfindungsgemäßen Verfahrens zu dem bereits bekannten Lightcycler-Verfahren besteht im Folgenden: Bei dem Lightcycler-Verfahren werden zwei Oligonukleotid-Sonden eingesetzt, die in räumlicher Nähe zueinander an das Amplifikat hybridisieren. Das über eine energetische Wechselwirkung (FRET) zwischen den beiden Sonden entstehende Fluoreszenzsignal wird anschließend detektiert. Im Lightcycler-Verfahren werden die beiden Sonden nur zusammen, nicht aber isoliert voneinander nachgewiesen. Bei dem erfindungsgemäßen Verfahren dagegen sind beide Sonden mit einem Farbstoff versehen, der eine isolierte Detektion ermöglicht. Dadurch können zwei Signale mit unterschiedlicher Aussagekraft generiert werden: das eine Signal steht für die Gesamt-DNA, das andere Signal spezifisch für die methylierte/unmethylierte DNA. Aus dem Verhältnis beider Signale kann dann der Methylierungsgrad an der untersuchten Position bestimmt werden. In besonders bevorzugten Ausführungsformen des erfindungsgemäßen Verfahrens werden Elemente der Lightcycler- und der Taqman-Technologie kombiniert. Dies erlaubt eine sehr leistungsfähige Quantifizierung.

Aufgrund der genannten besonderen biologischen und medizinischen Bedeutung der Cytosin-Methylierung und aufgrund der oben erwähnten Nachteile des Standes der Technik besteht ein großes technisches Bedürfnis an der Entwicklung leistungsfähiger Methoden zur quantitativen Methylierungsanalyse. Das erfindungsgemäße Verfahren stellt ein solches Verfahren zur Verfügung und stellt damit einen wichtigen technischen Fortschritt dar.

Gegenstand der vorliegenden Erfindung ist ein Verfahren zur Quantifizierung von Cytosinmethylierungen, das **dadurch gekennzeichnet, dass** folgende Schritte durchgeführt werden:

a) die zu untersuchende DNA wird so umgesetzt, dass 5-Methylcytosin unverändert bleibt, während unmethyliertes Cytosin in Uracil oder in eine andere Base umgewandelt wird, die sich im Basenpaarungsverhalten von Cytosin unterscheidet,
b) die umgewandelte DNA wird in Gegenwart zweier Real-Time-Sonden amplifiziert, wobei die eine Sonde spezifisch für entweder den methylierten oder den nichtmethylierten Zustand der untersuchten DNA ist, die andere Sonde methylierungsunspezifisch ist, eine der beiden Sonden einen Donorfarbstoff trägt, der den Farbstoff der anderen Sonde über einen FRET anregt, sobald die beiden Sonden an das Amplifikat hybridisieren, und die Sonde, die den Donorfarbstoff trägt, eine Taqman-Sonde ist, bei der der Farbstoff am 3'Ende und der Quencher in 5'Richtung von dem Farbstoff lokalisiert ist,
c) zu unterschiedlichen Zeitpunkten wird mittels einer Detektion der hybridisierten Sonden festgestellt, wieweit die Amplifikation fortgeschritten ist,
d) mit Hilfe der Signale wird der Methylierungsgrad der untersuchten DNA bestimmt.

Ein weiterer Gegenstand der vorliegenden Erfindung ist ein Kit zur Durchführung des erfindungsgemäßen Verfahrens, der aus zwei Primern, einer Polymerase und zwei Real-Time-Sonden besteht, wobei die eine Sonde spezifisch für entweder den methylierten oder den nichtmethylierten Zustand der untersuchten DNA ist, die andere Sonde methylierungsunspezifisch ist, eine der beiden Sonden einen Donorfarbstoff trägt, der den Farbstoff der anderen Sonde, die eine Lightcycler-Sonde ist, über einen FRET anregt, sobald die beiden Sonden an das Amplifikat hybridisieren, und die Sonde, die den Donorfarbstoff trägt, eine Taqman-Sonde ist, bei der der Farbstoff am 3'Ende und der Quencher in 5'Richtung von dem Farbstoff lokalisiert ist, sowie optional weitere für eine PCR erforderliche Reagenzien und/oder ein Bisulfitreagenz enthält.

Vorteilhafte Ausgestaltungen des erfindungsgemäßen Verfahrens und des erfindungsgemäßen Kits sind durch die abhängigen Ansprüche gekennzeichnet.

Beschreibung

Das erfindungsgemäße Verfahren eignet sich bevorzugt, aber nicht ausschließlich zur Methylierungsanalyse von DNA. Zur Methylierungsanalyse wird die DNA zunächst chemisch oder enzymatisch umgewandelt, bevorzugt durch eine Bisulfitumwandlung. Das Prinzip der Erfindung besteht dabei darin, dass eine Amplifikation in Gegenwart zweier Real-Time-Sonden durchgeführt wird. Die Sonden hybridisieren an dasselbe Amplifikat an unterschiedlichen Positionen.

In einer Ausführungsform bindet die eine Sonde methylierungsspezifisch an eine Methylierungsposition, während die andere Sonde unabhängig von Methylierungsstatus an die Amplifikate bindet. Anders als im bekannten Lightcyc-1er-Verfahren ist mindestens eine der beiden Sonden iso-1iert von der anderen detektierbar. Anschließend werden die Signale der Sonden gemessen. Aus dem Verhältnis der Signale zwischen spezifischer Sonde und unspezifischer Sonde lässt sich dann der Methylierungsgrad der untersuchten DNA bestimmen. Die Berechnung kann dabei über unterschiedliche Berechnungsmethoden erfolgen, etwa über die Signalintensitäten oder über die Ct-Werte.

Neben der Quantifizierung der DNA 1ässt sich das erfindungsgemäße Verfahren auch für weitere Anwendungen in der Methylierungsanalyse einsetzen. Werden zwei methylierungsspezifische Sonden verwendet, die an unterschiedlichen CpG-Positionen in der DNA binden, so lassen sich erfindungsgemäß diese beiden methylierten Positionen gegeneinander messen.

Darüber hinaus ist auch die gleichzeitige Untersuchung von Mutationen oder Polymorphismen und Methylierungen möglich, wenn eine Sonde verwendet wird, die spezifisch für die Mutation/ den Polymorphismus ist, und eine andere Sonde, die spezifisch für einen Methylierungsstatus ist.

Weiterhin ist das Verfahren auch außerhalb der Methylierungsanalyse einsetzbar. Eine Bisulfitumwandlung ist in diesem Fall nicht erforderlich. So können etwa Single Nukleotid Polymorphismen (SNP) quantifiziert werden, oder unterschiedliche SNP gegeneinander gemessen werden. Zudem kann das erfindungsgemäße Verfahren zur Quantifizierung allelspezifische Genexpression oder zur Untersuchung des Imprinting eingesetzt werden.

Besonders bevorzugt sind Ausführungsformen, in denen die beiden Real-Time-Sonden miteinander in Wechselwirkung treten. So ist eine besonders schnelle und effektive Quantifizierung möglich.

Das erfindungsgemäße Verfahren eignet sich zur Methylierungsanalyse. Bei einer bevorzugten Ausführungsform des erfindungsgemäßen Verfahrens handelt es sich um ein Verfahren zur Quantifizierung von Cytosinmethylierungen, das durch folgende Schritte gekennzeichnet ist:

a) die zu untersuchende DNA wird so umgesetzt, dass 5-Methylcytosin unverändert bleibt, während unmethyliertes Cytosin in Uracil oder in eine andere Base umgewandelt wird, die sich im Basenpaarungsverhalten von Cytosin unterscheidet,

b) die umgewandelte DNA wird in Gegenwart zweier Real-Time-Sonden amplifiziert, wobei die eine Sonde spezifisch für entweder den methylierten oder den nichtmethylierten Zustand der untersuchten DNA ist, während die andere Sonde methylierungsunspezifisch ist,

c) zu unterschiedlichen Zeitpunkten wird mittels einer Detektion der hybridisierten Sonden festgestellt, wieweit die Amplifikation fortgeschritten ist,

d) mit Hilfe der Signale wird der Methylierungsgrad der untersuchten DNA bestimmt.

Im ersten Schritt dieser Ausführungsform wird die zu untersuchende DNA mit einer Chemikalie oder mit einem Enzym so umgesetzt, dass 5-Methylcytosin unverändert bleibt, während unmethyliertes Cytosin in Uracil oder in eine andere Base umgewandelt wird, die sich im Basenpaarungsverhalten von Cytosin unterscheidet. Dabei kann die zu untersuchende DNA je nach diagnostischer oder wissenschaftlicher Fragestellung aus unterschiedlichen Quellen stammen. Für diagnostische Fragestellungen dienen als Ausgangsmaterial bevorzugt Gewebeproben, aber auch Körperflüssigkeiten, insbesondere Serum. Möglich ist auch, die DNA aus Sputum, Stuhl, Urin oder Gehirn-Rückenmarks-Flüssigkeit zu verwenden. Vorzugsweise wird die DNA zunächst aus der biologischen Probe isoliert. Die DNA-Extraktion erfolgt nach Standardmethoden, aus Blut etwa unter Verwendung des Qiagen UltraSens DNA Extraktions-Kits. Die isolierte DNA kann dann z.B. durch Umsatz mit Restriktionsenzymen fragmentiert werden. Die Reaktionsbedingungen und die in Frage kommenden Enzyme sind dem Fachmann bekannt und ergeben sich etwa aus den von den Herstellern mitgelieferten Protokollen. Anschließend wird die DNA chemisch oder enzymatisch umgewandelt. Bevorzugt erfolgt einen chemische Umsetzung mittels Bisulfit. Die Bisulfitumwandlung ist dem Fachmann in unterschiedlichen Variationen bekannt (siehe etwa: Frommer et al.: A genomic sequencing protocol that yields a positive display of 5-methylcytosine residues in individual DNA strands. Proc Natl Acad Sei U S A. 1992 Mar 1;89(5):1827-31; Olek, A modified and improved method for bisulphite based cytosine methylation analysis. Nucleic Acids Res. 1996 Dec 15; 24(24): 5064-6.; DE 100 29 915; DE 100 29 915). Besonders bevorzugt erfolgt die Bisulfitumwandlung in Gegenwart von denaturierenden Lösemitteln und eines Radikalfängers (vgl.: DE 100 29 915). Ganz besonders bevorzugte Ausführungsformen der Bisulfitumwandlung unter Verwendung von n-Alkylenglykolverbindungen, insbesondere von Diethylenglycoldimethylether (DME) oder von Dioxan oder Dioxanderivaten sowie unter Einsatz besondere Temperaturprofile und Aufreinigungsmethoden sind in der PCT-Anmeldung WO 2005/038051 beschrieben. In einer anderen bevorzugten Ausführungsform wird die DNA nicht chemisch, sondern enzymatisch umgewandelt. Dies ist etwa durch Einsatz von Cytidin-Deaminasen denkbar, die unmethylierte Cyidine schneller umsetzen als methylierte Cytidine. Ein entsprechendes Enzym ist kürzlich identifiziert worden (Bransteitter et al.: Activation-induced cytidine deaminase deaminates deoxycytidine on singlestranded DNA but requires the action of RNase. Proc Natl Acad Sci U S A. 2003 Apr 1; 100(7):4102-7).

Im zweiten Schritt des erfindungsgemäßen Verfahrens wird die umgewandelte DNA in Gegenwart zweier Real-Time-Sonden amplifiziert, wobei die eine Sonde spezifisch für entweder den methylierten oder den nicht-methylierten Zustand ist, während die andere Sonde methylierungsunspezifisch ist. Die erste Sonde wird im Folgenden als methylierungsspezifische Sonde bezeichnet. Bevorzugt erfolgt dabei eine Amplifikation mittels eines exponentiellen Amplifikationsverfahrens, besonders bevorzugt mittels einer PCR. Für die Amplifikation werden Primer verwendet, die spezifisch für die chemisch oder enzymatisch umgewandelte DNA sind. In einer bevorzugten Ausführungsform werden dabei methylierungsunspezifische Primer eingesetzt, d.h. Primer, die über keine CG- bzw. kein methylierungsspezifisches TG- oder CA-Dinukleotid verfügen. Mit diesen Primern erfolgt eine gleichmäßige Amplifikation methylierter und unmethylierter DNA. In einer anderen bevorzugten Ausführungsform werden methylierungsspezifische Primer verwendet, also Primer, die CG- bzw. methylierungsspezifische TG- oder CA-Dinukleotide tragen. So ist es möglich, einen größeren Sequenzbereich methylierungsspezifisch zu amplifizieren und dabei bestimmte Cytosinpositionen innerhalb dieser Sequenz mit Hilfe des erfindungsgemäßen Verfahrens zu quantifizieren. Das Design von methylierungsspezifischen und methylierungsunspezifischen Primern und die PCR-Reaktionsbedingungen gehören zum Stand der Technik (vgl.: etwa: US Patent 6,331,393; Trinh et al 2001, a.a.o.).

Die Amplifikation erfolgt in Gegenwart zweier unterschiedlicher Sonden, wobei die eine Sonde methylierungsspezifisch ist, während die andere Sonde spezifisch für den unmethylierten Zustand der DNA ist. Die methylierungsspezifische Sonde trägt dementsprechend mindestens ein CpG (im Falle des Nachweises methylierter DNA) ein spezifisches TG- bzw. CA-Dinukleotid (im Falle des Nachweises nicht-methylierter DNA). Bevorzugt tragen die Sonden drei spezifische Dinukleotide. Die methylierungsunspezifischen Sonden enthalten die entsprechenden Dinukleotide nicht. Die Sonden binden bevorzugt an unterschiedliche Positionen in der DNA. Bevorzugt verfügen beide Sonden über eine ähnliche Schmelztemperatur.

Bei den Sonden handelt es sich um Real-Time-Sonden. Hierunter werden im Folgenden Sonden verstanden, die es erlauben, die Amplifikate bereits während der Amplifikation nachzuweisen. Dem Fachmann sind unterschiedliche Real-Time-PCR-Varianten verwandt, etwa Lightcycler-, Taqman-, Sunrise-, Molecular Beacon- oder Eclipse-Sonden. Einzelheiten zum Aufbau und zum Nachweis dieser Sonden gehören zum Stand der Technik (vgl.: US Patent 6,331,393 mit weiteren Nachweisen). So kann das Design der Sonden etwa über die "PrimerExpress"-Software von Applied Biosystems (für Taqman-Sonden) oder über MGB Eclipse Design Software von Epoch Biosciences (für Eclipse-Sonden) erfolgen.

Bevorzugt tragen die beiden Sonden unterschiedliche Farbstoffe, so dass es möglich ist, beide Sonden getrennt voneinander zu detektieren. Die Amplifikation erfolgt bevorzugt zusammen mit beiden Sonden in einem Gefäß, so dass die Reaktionsbedingungen für beide Sonden identisch sind. Es ist allerdings nicht unbedingt erforderlich, dass die beiden Sonden unterschiedliche Markierungen trägen. Es ist auf der anderen Seite auch möglich, die Amplifikationen in unterschiedlichen Gefäßen durchzuführen. Hierdurch lassen sich störende Wechselwirkungen zwischen den Fluoreszenzfarbstoffen vermeiden.

In einer bevorzugten Ausführungsform binden die Amplifikate an unterschiedliche Stellen desselben Stranges des Amplifikats. In einer anderen bevorzugten Ausführungsform binden die Sonden jeweils an einen anderen Strang des Amplifikates (siehe im einzelnen unten).

Binden die Sonden an denselben Strang, so interagieren in einer bevorzugten Ausführungsform die Farbstoffe der beiden Sonden miteinander. So ist eine sehr leistungsfähige Quantifizierung möglich. Diese besonders bevorzugten Ausführungsformen sind unten detailliert beschrieben.

Im dritten Schritt des erfindungsgemäßen Verfahrens wird zu unterschiedlichen Zeitpunkten festgestellt, wieweit die Amplifikation fortgeschritten ist. Dies erfolgt über eine Detektion der an die Amplifikate gebundenen Sonden. Der Nachweis der Hybridisierungen findet während der einzelnen Amplifikationszyklen statt. Die Detektion erfolgt dabei in Abhängigkeit von den eingesetzten Sonden nach dem Stand der Technik.

Im vierten Schritt des erfindungsgemäßen Verfahrens wird mit Hilfe der detektierten Signale der Methylierungsgrad der untersuchten DNA bestimmt.

Die Bestimmung des Methylierungsgrades kann über unterschiedliche Weise geschehen. In einer bevorzugten Ausführungsform verfügen die Sonden über vergleichbare Längen und vergleichbare Schmelztemperaturen. Bevorzugt wird aus dem Verhältnis der Signalintensitäten der beiden Sonden der Methylierungsgrad der untersuchten DNA bestimmt. Dies kann etwa über die folgende Formel erfolgen:

$$M = 100 * I_{CG} / I_{UN}$$

Dabei handelt es sich bei ICG um die Signalintensität der für den methylierten Zustand spezifischen Sonde und bei IUN um die Signalintensität der methylierungsunspezifischen Sonde.

Besonders bevorzugt werden die Signalintensitäten während eines PCR-Zyklus in der exponentiellen Amplifikationsphase der PCR miteinander in Verhältnis gesetzt. Bevorzugt erfolgt eine Berechnung in der Nähe des Zyklus, bei dem die Amplifikation ihre maximale Steigung erreicht. Dies entspricht dem Wendepunkt der Fluoreszenzintensitätskurven bzw. des Maximums ihrer ersten Ableitung.

Die Berechnung erfolgt dabei zu einem Zeitpunkt, der bevorzugt bis zu fünf Zyklen vor oder nach dem Wendepunkt, besonders bevorzugt bis zu zwei Zyklen vor oder nach dem Wendepunkt und ganz besonders bevorzugt bis zu einem Zyklus vor oder nach dem Wendepunkt liegt. In der besten Ausführungsform findet die Berechnung direkt im Wendepunkt statt.

Die Bestimmung der Wendepunkte erfolgt bevorzugt über die erste Ableitung der Fluoreszenzintensitätskurven.

Vorzugsweise werden die Ableitungen zunächst einer Glättung unterzogen ("Spline", vgl.: Press, W. H., Teukolsky, S. A., Vetterling, W. T., Flannery, B. P. (2002). Numerical Recipes in C. Cambridge: University Press; Chapter 3.3.).

In einer anderen Ausführungsform erfolgt die Berechnung des Methylierungsgrades nicht über das Verhältnis der Fluoreszenzintensitäten, sondern über das Verhältnis von Schwellenwerten, bei denen eine gewisse Signalintensität überschritten wird, etwa bei Ct-Werten (s.o.). Die Bestimmung von Ct-Werten gehört zum Stand der Technik (vgl.: Trinh et al, a.a.o., 2002). Der Methylierungsgrad läßt sich dann über die folgende Formel bestimmen: Methylierungsgrad = $100/(1+2^{\Delta Ct})$.

Daneben ist es denkbar, andere Kriterien zur Berechnung des Methylierungsgrades zu verwenden, etwa die Fläche unter den Fluoreszenzkurven (area under the curve) oder die maximale Steigung der Kurven.

Eine Quantifizierung über die oben beschriebenen Verfahren ist besonders gut möglich, wenn die Assaybedingungen diesbezüglich zuvor optimiert wurden. Eine Optimierung erfolgt mit unterschiedlichen Methylierungsstandards (etwa mit 0%, 5%, 10%, 25%, 50%, 75% und 100% Methylierungsgrad). Als Standard wird bevorzugt DNA verwendet, die die gesamte genomische DNA oder einen repräsentativen Teil hiervon abdeckt. Die unterschiedlichen Methylierungsgrade erhält man durch entsprechende Mischungen aus methylierter und nicht-methylierter DNA. Die Herstellung methylierter DNA ist relativ einfach über die Verwendung der SssI-Methylase möglich. Dieses Enzym überführt im Sequenzkontext CG alle nicht-methylierten Cytosine in 5-Methylcytosin. Als vollständig nicht-methylierte DNA kann Sperma-DNA verwendet werden, die über einen nur geringen Methylierungsgrad verfügt (vgl.: Trinh et al. 2001, a.a.o.). Bevorzugt erfolgt aber die Herstellung nicht-methylierter DNA mittels einer sog. genomweiten Amplifikation (WGA - whole genome amplification, zur Übersicht: Hawkins et al.: Whole genome amplification--applications and advances. Curr. Opin. Biotechnol. 2002 Feb; 13(1): 65-7).WGA). Hierbei werden weite Teile des Genoms mittels "Random" oder degenerierter Primer amplifiziert. Da in der Amplifikation nur unmethylierte Cytosinnukleotide angeboten werden, resultiert nach mehreren Amplifikationszyklen eine vollständig unmethylierte DNA. Bevorzugt erfolgt dabei eine "Multiple Displacement Amplification" mittels der φ29 Polymerase (MDA, vgl.: Dean et al. 2002 a.a.o.; US Patent 6,124,120). Entsprechend hergestellte DNA ist über unterschiedliche kommerzielle Anbieter verfügbar ("GenomiPhi" von Amersham Biosciences, www4.amershambiosciences.com; "Repli-g" von Molecular Staging, www.molecularstaging.com). Indem der Quotient der Signale, die für den methylierten Zustand detektiert werden, und der Summe der Signale, die für den methylierten und den unmethylierten Zustand detektiert werden, gebildet wird, erhält man die gemessene Methylierungsrate. Trägt man diese gegen die theoretischen Methylierungraten (entsprechend dem Anteil methylierter DNA in den definierten Mischungen) auf und ermittelt die Regression, die durch die Messpunkte geht, erhält man eine Kalibrierungskurve. Bevorzugt erfolgt eine Kalibrierung mit unterschiedlichen Mengen an DNA, etwa mit 0,1; 1 und 10 ng DNA pro Ansatz.

Assays eignen sich besonders für die Quantifizierung über das erfindungsgemäße Verfahren, wenn die Kalibrierungskurven für den Zeitpunkt der exponentiellen Amplifikation möglichst über einen y-Achsenabschnitt bei Null verfügen. Benachbarte Methylierungszustände sollten mit einem hohen Fischer-Score (bevorzugt über 1, besonders bevorzugt über 3) unterschieden werden. Vorteilhaft ist weiterhin, wenn die über einen möglichst geringen y-Achsenabschnitt und einen möglichst hohen Fischer-Score (bevorzugt über 1, besonders bevorzugt über 3) verfügen. Vorteilhaft ist weiterhin, wenn die Kurven eine Steigung und eine Regression nahe dem Wert 1 haben.

Die Assays können diesbezüglich mittels Variation der Primer, der Sonden, des Temperaturprogramms und der weiteren Reaktionsparameter über Standardversuche optimiert werden.

Weitere bevorzugte Ausführungsformen zur Methylierungsanalyse

Wie oben ausgeführt, eignet sich das erfindungsgemäße Verfahren insbesondere zur Bestimmung des Methylierungsgrades der DNA, indem mittels einer methylierungspezifischen Sonde die methylierte DNA und mittels einer unspezifischen Sonde die Gesamt-DNA bestimmt wird. Die Erfindung ist jedoch nicht auf diese Anwendung beschränkt.

Vielmehr lässt sich das erfinderische Prinzip wie folgt formulieren: Die Amplifikation chemisch umgewandelter DNA wird mittels zweier Real-Time-Sonden detektiert. Die Sonden binden dabei innerhalb des Amplifikates an zwei verschiedene Positionen. Die Sonden tragen Farbstoffe, die eine isolierte Detektion mindestens einer der beiden Sonden ermöglichen. Aus dem Verhältnis der Signale kann auf das Verhältnis der Sequenzen, an die die Sonden hybridisieren, geschlossen werden. Dabei ist es bevorzugt, wenn eine der Sonden spezifisch für einen Methylierungsstatus ist, und die andere Sonde methylierungsunspezifisch an die Amplifikate bindet. Es ist daneben aber auch möglich, das Verhältnis von verschiedenen, benachbarten Cytosinpositionen zueinander zu bestimmen. Hierzu wird eine Sonde verwendet, die methylierungsspezifisch an die erste Position bindet, und eine zweite Sonde, die methylierungsspezifisch an die zweite Position bindet. Dabei ist es möglich, dass beide Sonden spezifisch für den methylierten Zustand sind. Auch ist es möglich, dass beide Sonden spezifisch für den unmethylierten Zustand sind. Denkbar ist auch, dass die eine Sonde spezifisch für den methylierten Zustand, und die andere Sonde spezifisch für den unmethylierten Zustand ist. Schließlich ist es auch möglich, das die Sonden auf unterschiedlichen Strängen des Amplifikates binden. In dieser Ausführungsform ist drauf zu achten, dass die Sonden nicht zueinander komplementär sind, da es dann zu einer Bildung von Sondendimeren kommt. Wenn die Sonden allerdings eine Überlappung von nur sehr wenigen Basen haben, ist es denkbar, dieselbe Position sowohl auf dem einen Strang wie auch auf dem anderen Strang zu detektieren (etwa auf dem einen Strang die methylierte Position, und auf dem anderen Strang die unmethylierte Position). Für diese Ausführungsform werden daher dieselben Positionen auf unterschiedlichen Strängen als unterschiedliche Positionen angesehen.

Dementsprechend lässt sich diese Ausführungsform des erfinderischen Verfahrens wie folgt beschreiben:

Verfahren zur Analyse von Cytosinmethylierungen, dadurch gekennzeichnet, dass folgende Schritte durchgeführt werden:

a) die zu untersuchende DNA wird so umgesetzt, dass 5-Methylcytosin unverändert bleibt, während unmethyliertes Cytosin in Uracil oder in eine andere Base umgewandelt wird, die sich im Basenpaarungsverhalten von Cytosin unterscheidet,
b) die umgewandelte DNA wird in Gegenwart zweier Real-Time-Sonden amplifiziert, wobei die Sonden spezifisch für entweder den methylierten oder den nicht-methylierten Zustand jeweils unterschiedlicher Cytosinpositionen sind,
c) zu unterschiedlichen Zeitpunkten wird mittels einer Detektion der hybridisierten Sonden festgestellt, wieweit die Amplifikation fortgeschritten ist,
d) mit Hilfe der Signale wird das Verhältnis der Methylierung an den beiden verschiedenen Positionen bestimmt.

Dabei erfolgen die Schritte a)-d) im Wesentlichen wie oben beschrieben. Auf die oben beschriebenen Ausführungen wird ausdrücklich verwiesen. Dementsprechend wird zunächst bevorzugt eine Bisulfitumwandlung durchgeführt, die besonders bevorzugt in Gegenwart von denaturierenden Lösemitteln und eines Radikalfängers (vgl.: DE 100 29 915) erfolgt. Ganz besonders bevorzugt erfolgt die Bisulfitumwandlung unter Verwendung von n-Alkylenglykolverbindungen, insbesondere von Diethylenglycoldimethylether (DME) oder von Dioxan oder Dioxanderivaten sowie unter Einsatz besondere Temperaturprofile und Aufreinigungsmethoden (WO 2005/03805).

Im zweiten Schritt des erfindungsgemäßen Verfahrens wird die umgewandelte in Gegenwart zweier Real-Time-Sonden amplifiziert, wobei beide Sonden methylierungsspezifisch sind, d.h. spezifisch entweder für den methylierten oder den nichtmethylierten Zustand der DNA sind. Die beiden Sonden sind dabei spezifisch für jeweils unterschiedliche Cytosinpositionen.

Die Amplifikation erfolgt besonders bevorzugt mittels einer PCR. Für die Amplifikation werden Primer verwendet, die spezifisch für die umgewandelte DNA sind. In einer bevorzugten Ausführungsform werden dabei methylierungsunspezifische Primer eingesetzt. In einer anderen bevorzugten Ausführungsform werden methylierungsspezifische Primer verwendet (s.o.) Das Design von methylierungsspezifischen und methylierungsunspezifischen Primern und die PCR-Reaktionsbedingungen gehören zum Stand der Technik (vgl.: etwa: US Patent 6,331,393; Trinh et al 2001, a.a.o.).

Die Amplifikation erfolgt in Gegenwart zweier unterschiedlicher methylierungsspezifischer Sonden. Die Sonden tragen dementsprechend mindestens ein CpG (im Falle des Nachweises methylierter DNA) oder ein spezifisches TG- bzw. CA-Dinukleotid (im Falle des Nachweises nicht-methylierter DNA). Bevorzugt tragen die Sonden drei spezifische Dinukleotide. Bevorzugt verfügen beide Sonde über eine ähnliche Schmelztemperatur.

Bei den Sonden handelt es sich um Real-Time-Sonden. Hierunter werden Sonden verstanden, die es erlauben, die Amplifikate bereits während der Amplifikation nachzuweisen. Dem Fachmann sind unterschiedliche Real-Time-PCR-Varianten verwandt, etwa Lightcycler-, Taqman-, Sunrise-, Molecular Beacon- oder Eclipse-Sonden. Einzelheiten zum Aufbau und zum Nachweis dieser Sonden gehören zum Stand der Technik (vgl.: US Patent 6,331,393 mit weiteren Nachweisen). So kann das Design der Sonden etwa über die "PrimerExpress"-Software von Applied Biosystems (für Taqman-Sonden) oder über MGB Eclipse Design Software von Epoch Biosciences (für Eclipse-Sonden) erfolgen.

Bevorzugt tragen die beiden Sonden unterschiedliche Farbstoffe, so dass es möglich ist, zumindest eine der beiden Sonden unabhängig von der anderen zu detektieren. Die Amplifikation erfolgt bevorzugt zusammen mit beiden Sonden in einem Gefäß, so dass die Reaktionsbedingungen für beide Sonden identisch sind. Es ist allerdings nicht unbedingt erforderlich, dass die beiden Sonden unterschiedliche Markierungen tragen. Es ist auf der anderen Seite auch möglich, die Amplifikationen in unterschiedlichen Gefäßen durchzuführen. Hierdurch lassen sich störende Wechselwirkungen zwischen den Fluoreszenzfarbstoffen vermeiden.

In besonders bevorzugten Ausführungsformen interagieren die Farbstoffe der beiden Sonden miteinander. So ist eine sehr leistungsfähige Quantifizierung möglich. Diese besonders bevorzugten Ausführungsformen sind unten detailliert beschrieben.

Im dritten Schritt des erfindungsgemäßen Verfahrens wird zu unterschiedlichen Zeitpunkten festgestellt, wieweit die Amplifikation fortgeschritten ist. Dies erfolgt über eine Detektion der an die Amplifikate gebundenen Sonden. Der Nachweis der Hybridisierungen findet während der einzelnen Amplifikationszyklen statt. Die Detektion erfolgt dabei in Abhängigkeit von den eingesetzten Sonden nach dem Stand der Technik.

Im vierten Schritt des erfindungsgemäßen Verfahrens wird mit Hilfe der detektierten Signale das Verhältnis der Methylierung an den beiden verschiedenen Positionen bestimmt.

Diese Berechnung kann wie oben detailliert beschrieben auf unterschiedliche Weise geschehen. In einer bevorzugten Ausführungsform erfolgt die Berechnung über das Verhältnis der Signalintensitäten. Dies erfolgt besonders bevorzugt während eines PCR-Zyklus in der exponentiellen Amplifikationsphase der PCR. Bevorzugt erfolgt eine Berechnung in der Nähe des Zyklus, bei dem die Amplifikation ihre maximale Steigung erreicht. Dies entspricht dem Wendepunkt der Fluoreszenzintensitätskurven bzw. des Maximums ihrer ersten Ableitung. Die Berechnung erfolgt dabei zu einem Zeitpunkt, der bevorzugt bis zu fünf Zyklen vor oder nach dem Wendepunkt, besonders bevorzugt bis zu zwei Zyklen vor oder nach dem Wendepunkt und ganz besonders bevorzugt bis zu einem Zyklus vor oder nach dem Wendepunkt liegt. In der besten Ausführungsform findet die Berechnung direkt im Wendepunkt statt. Für den Fall, dass die Wendepunkte der beiden Kurven in unterschiedlichen Zyklen liegen, erfolgt die Berechnung bevorzugt bei dem Wendepunkt der Kurve, die zu diesem Zeitpunkt das höchste Signal aufweist. Die Wendepunkte werden wie oben beschrieben bestimmt.

In einer anderen Ausführungsform erfolgt die Berechnung des Methylierungsgrades nicht über das Verhältnis der Fluoreszenzintensitäten, sondern über das Verhältnis von Schwellenwerten, bei denen eine gewisse Signalintensität überschritten wird, etwa bei Ct-Werten (s.o.).Daneben ist es denkbar, andere Kriterien zur Berechnung des Methylierungsgrades zu verwenden, etwa die Fläche unter den Fluoreszenzkurven (area under the curve) oder die maximale Steigung der Kurven.

Eine Quantifizierung über die oben beschriebene Verfahren ist besonders gut möglich, wenn die Assaybedingungen diesbezüglich zuvor optimiert wurden (s.o.). Assays eignen sich besonders für die Quantifizierung über das erfindungsgemäße Verfahren, wenn die Kalibrierungskurven für den Zeitpunkt der exponentiellen Amplifikation möglichst über einen y-Achsenabschnitt bei Null verfügen. Benachbarte Methylierungszustände sollten mit einem hohen Fischer-Score (bevorzugt über 1, besonders bevorzugt über 3) unterschieden werden. Vorteilhaft ist weiterhin, wenn die über einen möglichst geringen y-Achsenabschnitt und einen möglichst hohen Fischer-Score (bevorzugt über 1, besonders bevorzugt über 3) verfügen. Vorteilhaft ist weiterhin, wenn die Kurven eine Steigung und eine Regression nahe dem Wert 1 haben (s.o.).

Weitere bevorzugte Ausführungsformen zur gleichzeitigen Untersuchung von Methylierungen und Mutationen /SNP

Wie oben ausgeführt, eignet sich das erfindungsgemäße Verfahren insbesondere zur Methylierungsanalyse. Darüber hinaus kann es aber auch für eine gleichzeitige Analyse von Methylierungen und Mutationen bzw. Polymorphismen eingesetzt werden. Dabei wird die DNA zunächst bisulfitumgewandelt, und danach mit Hilfe zweier Real-Time-Sonden amplifiziert. Die eine Sonde ist dabei spezifisch für eine bestimmte Methylierungsposition, während die andere Sonde spezifisch für eine SNP-Position ist. Dementsprechend lässt sich diese Ausführungsform des erfinderischen Verfahrens wie folgt beschreiben:

Verfahren zur gleichzeitigen Analyse von Cytosinmethylierungen und Mutationen/SNP, **dadurch gekennzeichnet, dass** folgende Schritte durchgeführt werden:

a) die zu untersuchende DNA wird so umgesetzt, dass 5-Methylcytosin unverändert bleibt, während unmethyliertes Cytosin in Uracil oder in eine andere Base umgewandelt wird, die sich im Basenpaarungsverhalten von Cytosin unterscheidet,
b) die umgewandelte DNA wird in Gegenwart zweier Real-Time-Sonden amplifiziert, wobei eine der Sonden spezifisch für entweder den methylierten oder den nichtmethylierten Zustand der DNA ist, und die andere Sonde spezifisch für eine Mutation/ einen Polymorphismus ist.
c) zu unterschiedlichen Zeitpunkten wird mittels einer Detektion der hybridisierten Sonden festgestellt, wieweit die Amplifikation fortgeschritten ist,
d) mit Hilfe der Signale werden die Verhältnisse zwischen der Methylierung und der Mutation/ dem Polymorphismus bestimmt.

Dabei erfolgt die Schritte a)-d) im Wesentlichen wie oben beschrieben. Auf die oben beschriebenen Ausführungen wird ausdrücklich verwiesen. Es werden dabei bevorzugt Sonden eingesetzt, von denen sich zumindest eine isoliert von der anderen nachweisen lässt.

Besonders bevorzugte Ausführungsformen des erfindungsgemäßen Verfahrens durch Verwendung interagierender Sonden

In besonders bevorzugten Ausführungsformen ist das erfindungsgemäße Verfahren so ausgestaltet, dass das die beiden eingesetzten Real-Time-Sonden über ihre Farbstoffe in einer besonderen Form miteinander interagieren. Bereitsdie herkömmlichen Lightcycler und Taqman-Verfahren nutzen eine Interaktion zwischen unterschiedlichen Farbtoffen. So wird bei dem Taqman-Verfahren eine Sonde verwendet, die sowohl einen Farbstoff wie auch einen Quencher trägt. Im Zuge der Amplifikation wird die Sonde abgebaut. Damit werden Quencher und Farbstoff voneinander getrennt, so dass ein Signal detektiert werden kann. Beim Lightcycler-Verfahren hybridisieren dagegen zwei Sonden in unmittelbar Nachbarschaft, wobei der Farbstoff der einen Sonde über einen Energietransfer (FRET) angeregt wird. Das erfindungsgemäße Verfahren kombiniert nun beide Prinzipien und ermöglicht so eine sehr leistungsfähige Analyse insbesondere von Cytosinmethylierungen.

Bevorzugt werden dabei gleichzeitig eine Taqman und eine Lightcycler-Sonde verwendet, die in unmittelbarer Nähe zueinander hybridisieren. Dabei dient der Fluoreszenz-farbstoff der Taqman-Sonde als Donor zur Anregung der Lightcycler-Sonde. Daher entstehen mindestens zwei unterschiedliche Signale: Das Signal der Taqman-Sonde bei ihrer Degradation und das Signal der Lightcycler-Sonde bei Hybridisierung in der Nähe der Taqman-Probe. Bei dem herkömmlichen Lightcyler-Verfahren entsteht dagegen nur ein Signal.

In einer besonders bevorzugten Ausführungsform ist die Taqman-Sonde dabei in besondere Form strukturiert: Üblicherweise liegt der Fluoreszenz-Farbstoff am 5'Ende und der Quencher am 3'-Ende. Erfindungsgemäß wird der Farbstoff stattdessen am 3'Ende positioniert. Der Quencher in 5' Richtung von dem Farbstoff lokalisiert, liegt also etwa in der Mitte oder am 5'-Ende der Sonde. Die Sequenz der Taqman-Sonde ist bevorzugt methylierungsunspezifisch, d.h. sie enthält weder CG-Dinukleotide noch methylierungsspezifische TG- oder CA-Dinukleotide. Die Taqman-Sonde hybridisiert daher unabhängig von dem ursprünglichen Methylierungsstatus an die Bisulfit-DNA und dient daher der Bestimmung der Gesamtmenge an Bisulfit-DNA. Daneben wird eine Lightcycler-Sonde verwendet, deren Sequenz methylierungsspezifisch ist, d.h. mindestens ein methylierungsspezifisches CG-, TG-, oder CA-Dinukleotid enthält. Die Lightcycler-Sonde dient zur Messung des methylierten bzw. unmethylierten Anteils der DNA. Die Lightcycler-Sonde ist so konstruiert, dass sie in der Nähe der Taqman-Sonde an die Amplifikate bindet. Der am 3'-Ende der Taqman-Sonde lokalisierte Farbstoff kann dann über einen FRET den Farbstoff der Lightcyler-Sonde anregen. Die Signale der Taqman- und Lightcycler-Sonde können dann in unterschiedlichen Kanälen gemessen werden. Aus dem Verhältnis der beiden Signale kann dann der Methylierungsgrad an der untersuchten Position bestimmt werden. Diese Ausführungsform hat den Vorteil, dass das spezifische Methylierungssignal durch die Lightcycler-Sonde nur dann generiert wird, wenn das methylierungsunspezifische Signal durch die Taqman-Sonde bereits entstanden ist. Dies führt zu einer erhöhten Spezifität des Methylierungsnachweises.

Diese besonders bevorzugte Ausführungsform des erfindungsgemäßen Verfahrens zur Quantifizierung von Cytosinmethylierungen, lässt sich durch folgende Schritte beschreiben:

a) die zu untersuchende DNA wird so umgesetzt, dass 5-Methylcytosin unverändert bleibt, während unmethyliertes Cytosin in Uracil oder in eine andere Base umgewandelt wird, die sich im Basenpaarungsverhalten von Cytosin unterscheidet,
b) die umgewandelte DNA wird in Gegenwart zweier Real-Time-Sonden amplifiziert, wobei mindestens eine der Sonden isolierte detektierbar ist, und wobei die eine Sonde spezifisch für entweder den methylierten oder den nichtmethylierten Zustand der untersuchten DNA ist, während die andere Sonde methylierungsunspezifisch ist,
c) die methylierungsunspezifische Sonde einen Donorfarbstoff trägt, der den Farbstoff der methylierungsspezifischen Sonde über einen FRET anregt, sobald sie nebeneinander an das Amplifikat hybridisieren,
d) zu unterschiedlichen Zeitpunkten wird mittels einer Detektion der hybridisierten Sonden festgestellt, wieweit die Amplifikation fortgeschritten ist,
e) mit Hilfe der Signale wird der Methylierungsgrad der untersuchten DNA bestimmt.

Probenaufarbeitung, Bisulfit-Umwandlung, Amplifikation und Auswertung der Signale erfolgen dabei wie im Detail oben beschrieben.

Bevorzugt wird dabei in Schritt c) als methylierungsunspezifische Sonde eine Taqman-Sonde benutzt, bei der Farbstoff am 3'Ende, und der Quencher in 5' Richtung von dem Farbstoff lokalisiert ist. Weiterhin wird bevorzugt als methylierungsspezifische Sonde eine Lightcyler-Sonde verwendet, deren Farbstoff von dem Farbstoff der Taqman-Sonde angeregt wird.

Diese besonders bevorzugte Ausführungsform des erfindungsgemäßen Verfahrens kann auch eingesetzt werden, um das Verhältnis zweier unterschiedlicher Methylierungspositionen zueinander zu bestimmen. Diese Anwendung ist allgemein bereits oben beschrieben. Auf die Ausführungen wird ausdrücklich verwiesen. In dieser besonders bevorzugten Ausführungsform werden dann zwei methylierungsspezifische Sonden verwendet, wobei die eine Sonde die andere Sonde über einen FRET anregt.

Diese besonders bevorzugte Ausführungsform des erfindungsgemäßen Verfahrens kann auch zur gleichzeitigen Untersuchung von Mutationen/Polymorphismen und Methylierung sowie außerhalb der Methylierungsanalyse eingesetzt werden, etwa zur Quantifizierung von Polymorphismen/Mutationen oder zur Analyse der allelspezifischen Genexpression. Diese Anwendungen sind im Detail bereits oben beschrieben. In dieser besonders bevorzugten Ausführungsform werden zwei Sonden verwendet, die wie oben beschrieben miteinander interagieren, etwa in einer Lightcycler-Taqman-Kombination.

Einsatz der erfindungsgemäßen Verfahren

Eine besonders bevorzugte Verwendung der erfindungsgemäßen Verfahren insbesondere zur Methylierungsanalyse liegt in der Diagnose oder Prognose von Krebserkrankungen oder anderen mit einer Veränderung des Methylierungsstatus assoziierten Krankheiten. Hierzu gehören u.a. CNS-Fehlfunktionen, Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion. Das erfindungsgemäße Verfahren eignet sich außerdem zur Vorhersage von unerwünschten Arzneimittelwirkungen und zur Unterscheidung von Zelltypen oder Geweben oder zur Untersuchung der Zelldifferenzierung.

Erfindungsgemäße Kits

Weiterhin ist erfindungsgemäß auch ein Kit zur Durchführung des erfindungsgemäßen Verfahrens, der aus zwei Primern, einer Polymerase und zwei Real-Time-Sonde besteht, sowie optional weitere für eine PCR erforderliche Reagenzien und/oder eine Bisulfitreagenz enthält.

Von den beiden Real-Time-Sonden bindet mindestens eine Sonde methylierungs-/SNP- oder allelspezifisch an die nachzuweisende Position, während die andere Sonde methylierungs-/SNP- oder allelspezifisch oder methylierungs-/SNP- oder allelunspezifisch an die zu untersuchende Sequenz bindet. Im Gegensatz zu dem bekannten Lightcycler-Verfahren ist dabei mindestens eine der Sonde isoliert von der anderen detektierbar. Bevorzugt interagieren die Farbstoffe der Sonden miteinander. Bevorzugt handelt es sich bei der einen Sonde um eine Lightycler-Sonde und bei der anderen Sonde um eine Taqman-Sonde. Bevorzugt ist die Taqman-Sonde so aufgebaut, dass der Farbstoff am 3'Ende, und der Quencher in 5' Richtung von dem Farbstoff lokalisiert ist. Weiterhin wird bevorzugt als methylierungsspezifische Sonde eine Lightcyler-Sonde verwendet, deren Farbstoff von dem Farbstoff der Taqman-Sonde angeregt wird.

**Beispiele**

Beispiel 1:

**Dual-Color Real Time PCR zur Detektion methylierter DNA im IGF2-Gen der Maus bei gleichzeitiger Detektion der Gesamt-DNA durch Anwendung des erfindungsgemäßen Verfahrens**

Im vorliegenden Beispiel wurde eine Real Time PCR zur Detektion von methylierter bisulfit-konvertierter DNA durchgeführt. Die chromosomale Maus-DNA (Promega Corporation) wurde mittels Sss I Methyltransferase genomweit methyliert. Unmethylierte Kontroll-DNA wurde durch genomweite Amplifikation mittels phi29 Polymerase (GenomiPhi-Kit, Amersham) hergestellt. Methylierte und unmethylierte DNA wurden mit Natrium-Bisulfit behandelt und anschließend mit Natriumhydroxid desulfoniert (Verfahren nach Patent WO 2005/038051). Mittels bisulfit-spezifischer Primer (SeqID-1, SeqID-2) wurde eine 161 bp lange Sequenz des IGF2-Gens der Maus (SeqID-5, NT_039437.4, nt909691 - nt909851) im LightCycler-System amplifiziert. Die Verwendung einer am 5'-Fluoreszein- und 3'-BHQ1-modifizierten Sonde (SeqID-3) diente der Detektion der Gesamt-DNA im Kanal Ch1 (530 nm). Dabei wird das Signal durch Exonuklease-Aktivität der Polymerase erzeugt, die das 5'-Ende der Sonde hydrolysiert und damit Fluoreszein-Farbstoff freisetzt. Die Sequenz der TaqMan-Sonde wurde so gewählt, dass sie keine CG-Dinukleotide enthält, also an die Gesamt-DNA unabhängig vom Methylierungsstatus bindet. Der Reaktion enthielt weiterhin eine zweite Sonde (SeqID-4) die in direkter Nachbarschaft zur ersten Sonde (SeqID-3) auf dem gleichen DNA-Strang bindet. Sie überspannt 3 CpGs und diente dem Nachweis methylierter DNA der IGF2-Sequenz. Das 3'-Ende der zweiten Sonde(SeqID-4) wurde mit dem LightCycler Farbstoff LCred640 zur Detektion bei einer Wellenlänge von 640 nm modifiziert. Die zeitgleiche Bindung der beiden Sonden in der Annealingphase der PCR erzeugt durch Fluoreszenz-Resonanz-Energie-Transfer (FRET) ein Signal im Kanal Ch2 (640 nm). Im Experiment wurden 100ng unmethylierte bisulfit-konvertierte DNA im Kanal Ch1 (530 nm) mit einem CT (Signaldurchbruchszyklus, engl. cycle threshold) von 26,75 gemessen. 1 ng methylierte DNA wurde im gleichen Kanal bei CT 36,25 detektiert (Abbildung 1). Im Kanal Ch2 hingegen wurde ausschließlich methylierte DNA mit einem CT 36,25 detektiert (Abbildung 2). Alle CT sind Durchschnittswerte aus 2 Wiederholungen (Tabelle 2). Im Folgenden werden die Reaktionsbedingungen und Ergebnisse im Einzelnen beschriebenen.

Die PCR Reaktionen erfolgten im LightCycler in 20µl Reaktionsvolumen und enthielten:

- 10 µl of Templat-DNA
- 2µl PCR buffer (Qiagen, enthält 1,5mM MgCl2)
- 0,25 mg/ml BSA (Sigma, non acetylated)
- 0,25 mM dNTPs each (dATP, dTTP, dGTP, CTP, Fermentas)
- 3.0 mM $MgCl_2$ (Qiagen)
- 0.30 µM Forwardprimer (SeqID-1, TIB-MolBiol)
- 0.30 µM Reverseprimer (SeqID-2, TIB-MolBiol)
- 0.15 µM Probe1 (SeqID-3, TIB-MolBiol)
- 0.15 µM Probe2 (SeqID-4, TIB-MolBiol)
- 2 Units HotStarTaq Polymerase (Qiagen)

Das Temperatur-Zeit-Profil für wurde wie folgt programmiert:

- Aktivierung der Polymerase: 15 min bei 95˚C

- 55 Temperaturzyklen: 10 sec bei 95˚C
  30 sec bei 56˚C
  10 sec bei 72˚C

Abschließend wird die Reaktion auf 35˚C abgekühlt. Die Auswertung erfolgte mit der SoFar Software 1.1.1 (© 2005 Jochen Wilhelm).

**Tabelle 1:** Sequenzen

| SeqID | Name | Sequence |
|---|---|---|
| SeqID-1 | IGF2-b2-F | TtAtTGATGGTTGtTGGAtATtTt |
| SeqID-2 | IGF2-b2-R | aAaaCCTaCCTaCCCTCCTa |
| SeqID-3 | IGF2_Fluo | fluo-TGGttTtTtTGAAtTtTTTGAGtTtTTTG-BHQ1 |
| SeqID-4 | IGF2_Red | CGATtAGGGGACGATGACG-red640 |
| SeqID-5 | IGF2-amp | TtAtTGATGGTTGtTGGAtATtTtCGAAGAGGtTttttCGTGGGCGGGGTtTTTGGGTGGTAAtACGATtAGGGGACGATGACGTTTGGttTtTtTGAAtTtTTTGAGtTtTTTGGtAAG-tATGCGAtttCGGCGGGtACGtAG-GAGGGtAGGtAGGttTt |

Die Abkürzungen bedeuten:

Fluo = Fluoreszein,
red640 = LightCycler Farbstoff für 640 nm,
BHQ1 = BlackHoleQuencherl.
Kleine Buchstaben weisen auf Cytosin-Positionen, die durch Bisulfit-Konversion in Uracil umgewandelt bzw. anschließend in der PCR durch Thymin ersetzt werden.

**Tabelle 2:** In der Real Time PCR gemessenen CT (threshold cycle)

| DNA | CT in Kanal Ch1 (530 nm) | CT in Kanal Ch2 (640 nm) |
|---|---|---|
| 1ng methylierte bisul-fit-konvertierte DNA | 36,25 | 36,25 |
| 100ng nicht methylierte bisulfit-konvertierte DNA | 26,75 | - |
| Negativ Kontrolle (Wasser) | - | - |

Beispiel 2:

**Allelspezifische Quantitative Methylierungsmessung mittels Real Time PCR**

Das Beispiel beschreibt die Messung des Anteils methylierter DNA im einer Region des IGF2-Gens der Maus mittles allelspezifischem QM-Assay. Die QM-Assay Technologie basiert auf der gemeinsamen Amplifikation methylierter und unmethylierter bisulfit-konvertierter DNA in einer Real Time PCR, wobei die Detektion mit zwei unterschiedlich markierten Sonden methylierungsspezifisch erfolgt. Die Signale für methylierte und unmethylierte DNA werden bei unterschiedlichen Wellenlängen detektiert. Mittels Kalibrierung der Messwerte anhand definierter DNA-Mischungen aus methylierter und unmethylierter DNA kann der prozentuale Anteil methylierter DNA für die untersuchte Region errechnet werden. Das vorliegende Beispiel demonstriert, dass durch den Einsatz einer zweiten allelspezifischen Sonde (SeqID-8 und SeqID-9) die Messung der Methylierung auf jeweils eines von zwei Allelen beschränkt werden kann (Abbildung 3). Dazu wurde eine stammspezifische Donor-Sonde entwickelt, die die Polymorphismen eines Labor-Mausstammes (SD7) überspannt (SeqID-9). Es handelte sich dabei um Basenaustausche (SNPs, single nucleotide polymorphism) an den Positionen nt909785 (T -> A) sowie nt909792 (T -> G) der Sequenz NT_039437.4. Die SD7-spezifische Donor-Sonde hybridisiert neben den methylierungsspezifischen Akzeptor-Sonden (SeqID-10, Seq ID -11) und erzeugt ein FRET-Signal (Fluoreszenz-Resonanz-Energie-Transfer). Je nach Methylierungsstatus bindet die Akzeptor-Sonde für methylierte (SeqID-10) oder die Akzeptor-Sonde für unmethylierte DNA (SeqID-11). Die bei unterschiedlichen Wellenlängen gemessenen Signale werden nach dem Verfahren des QM Assay ausgewertet. Die ermittelte Methylierungsrate bezieht sich in diesem Fall nur auf die DNA des Laborstammes (SD7).

Nach dem gleichen Prinzip wurde ein zweiter Assay spezifisch für die Methylierung des Wildstammes (BL6) realisiert. Die verwendete Donor-Sonde (SeqID-8) bindet dabei an der selben Position, enthält jedoch an den Positionen der Polymorphismen, die für den Wildstamm spezifischen Basen.

Durch die kontrollierte Kreuzung des Wildstammes (BL6) mit dem Laborstamm (SD7) entstehen hetereozygote Nachkommen deren Chromosomen anhand der beschrieben Polymorphismen unterschieden und dem jeweiligen Elterntier und damit Geschlecht zugeordnet werden können. Die in diesem Beispiel untersuchte DNA wurde durch Professor Jörn Walter von der Universität des Saarlandes zur Verfügung gestellt.

Die DNA wurde wie im Beispiel 1 beschrieben bisulfitkonvertiert.

In beiden QM Assays wurden bisulfit-spezifischer Primer (SeqID-6, SeqID-7) benutzt die eine 161 bp lange Sequenz des IGF2-Gens der Maus (SeqID-12 bzw. Seq-ID-13, NT_039437.4, nt909691 - nt909851) amplifizieren. Der SD7-spezifische QM Assay wurde mit Mischungen von SD7-DNA der BL6-spezifische QM Assay mit Mischungen von BL6-DNA kalibriert. Die vollständig methylierte und unmethylierte DNA wurde mittels *SssI* Methyltransferase bzw. durch genomweite Amplifikation mit phi29 Polymerase hergestellt (siehe Beispiel 1). Die Ergebnisse der Kalibrierung des SD7-QM Assays sind in Abbildung 4, die des BL6-QM Assay in Abbildung 5 dargestellt.

Aus Mäusen mit dem Kreuzungstyp BL6xSD7 sowie SD7xBL6 (erstgenannter Stamm ist jeweils weiblichen Geschlechts) wurde DNA von unterschiedlichem Gewebetypen gewonnen. 10 ng bisulfit-konvertierter Maus-DNA wurden in 2 Wiederholungen mit dem BL6-spezifischen und anschließend auch dem SD7-spezifischen QM Assay vermessen. Die ermittelten CT für die Signale von methylierter und unmethylierter DA sind in Tabelle 3 zusammengefasst. Aus den CT wurden mit Hilfe der Kalibrierung (Abbildung 4 und 5) die Methylierungssraten für das jeweilige Allel berechnet (Tabelle 4 und 5). Es konnte gezeigt werden, dass für alle untersuchten Gewebetypen jeweils nur das parental (väterlich) vererbte Chromosom Methylierung aufweist, während im maternal (mütterlich) vererbten Chromosom keine Methylierung nachweisbar war. Für Herz, Darm und Nieren-Gewebe konnten Daten für reziproke Kreuzungen ermittelt werden, die jeweils gute Übereinstimmung aufweisen: Herz 21% und 24%, Darm 25% und 35%, Niere 13% und 23%. Für Gehirn- und Lebergewebe lag jeweils nur der Kreuzungstyp SD7 x BL6 vor. Die gemessenen Methylierungsraten ergaben für Gehirn 3% und für Leber 55%. Eine Übersicht aller Ergebnisse geben die Diagramme in Abbildung 6.

Im Folgenden werden die Reaktionsbedingungen im Einzelnen beschriebenen. Die PCR Reaktionen erfolgten im LightCycler in 20μl Reaktionsvolumen und enthielten:

- 10 μl of Templat-DNA

- 2µl PCR buffer (Qiagen, enthält 1,5mM MgCl2)
- 0,25 mg/ml BSA (Sigma, non acetylated)
- 0,25 mM dNTPs each (dATP, dTTP, dGTP, CTP, Fermentas)
- 3.0 mM $MgCl_2$ (Qiagen)
- 0.30 µM Forwardprimer (SeqID-6, TIB-MolBiol)
- 0.30 µM Reverseprimer (SeqID-7, TIB-MolBiol)
- 0.15 µM Probe1 (SeqID-8 oder SeqID-9, TIB-MolBiol)
- 0.15 µM Probe2 (SeqID-10, TIB-MolBiol)
- 0.15 µM Probe3 (SeqID-11, TIB-MolBiol)
- 2 Units HotStarTaq Polymerase (Qiagen)

Das Temperatur-Zeit-Profil für wurde wie folgt programmiert:

- Aktivierung der Polymerase: 15 min bei 95°C

- 50 Temperaturzyklen: 10 sec bei 95°C
  30 sec bei 56°C
  10 sec bei 72°C

Die Auswertung erfolgte mit der SoFar Software 1.1.1 (© 2005 Jochen Wilhelm). Die ermittelten CT wurden nach dem Prinzip des QM Assay ausgewertet. Die Ergebnisse sind Durchschnittswerte aus 2 Wiederholungen (Tabelle 4 und 5).

**Tabelle 3:** Sequenzen

| SeqID | Name | Sequence |
|---|---|---|
| SeqID-6 | IGF2-b2-F | TtAtTGATGGTTGtTGGAtATtTt |
| SeqID-7 | IGF2-b2-R | aAaaCCTaCCTaCCCTCCTa |
| SeqID-8 | IGF2-BL6-Fluo | TGGttTtTtTGAAtTtTTTGAGtTtTTTG-Fluo |
| SeqID-9 | IGF2-SD7-Fluo | TGGttTtAtTGAAtGtTTTGAGtTtTTTG-Fluo |
| SeqID-10 | IGF2_Red6 | red640-tAAGtATGCGAtttCGGCGG-Pho |
| SeqID-11 | IGF2_Red7 | red705-tAAGtATGtGAttttGGtGGGtAt-Pho |
| SeqID-12 | IGF2-amp-BL6 | TtAtTGATGGTTGtTGGAtATtTtCGAAGAG GtTttttCGTGGGCGGGGTtTTTGGGTGGTA AtACGATtAGGGGACGATGACGTTTGGttTt TtTGAAtTtTTTGAGtTtTTTGGtAAGtATG CGAtttCGGCGGGtACGtAG-GAGGGtAGGtAGGttTt |

(fortgesetzt)

| SeqID | Name | Sequence |
|---|---|---|
| SeqID-13 | IGF2-amp-SD7 | TtAtTGATGGTTGtTGGAtATtTtCGAAGAG<br>GtTttttCGTGGCGGGGTtTTTGGGTGGTA<br>AtACGATtAGGGGACGATGACGTTTGGttTt<br>AtTGAAtGtTTTGAGtTtTTTGGtAAGtATG<br>CGAtttCGGCGGGtACGtAG-<br>GAGGGtAGGtAGGttTt |

Die Abkürzungen bedeuten:

Fluo=Fluoreszein,
red640=LightCycler Farbstoff für 640 nm,
red705=LightCycler Farbstoff für 705 nm,
Pho=3'-Phosphat-Modifikation.
Kleine Buchstaben weisen auf Cytosin-Positionen, die durch Bisulfit-Konversion in Uracil umgewandelt bzw. anschließend in der PCR durch Thymin ersetzt werden. Die unterstrichenen Positionen kennzeichnen zwei Polymorphismen im SD7 Labor-Mausstamm an den Positionen nt909785 (T -> A) sowie nt909792 (T -> G).

**Tabelle 4:** Ergebnisse des BL6-QM Assay. Gemessene CT für verschiedene Gewebetypen aus unterschiedlichen Kreuzungstypen sowie die daraus ermittelten Methylierungsraten.

| Kreuzungstyp | Gewebe | % Methylierung (Mittelwert) | | CT 640nm (CG-Sonde) | CT 705nm (tG-Sonde) | Ratio | % Methylierung (Einzelwerte) |
|---|---|---|---|---|---|---|---|
| BL6xSD7 | Herz | **0** | 1. Messung | >50 | 28,88 | 0 | 0 |
| | | | 2. Messung | >50 | 29,02 | 0 | 0 |
| BL6xSD7 | Darm | **0** | 1. Messung | >50 | 29,5 | 0 | 0 |
| | | | 2. Messung | >50 | 29,4 | 0 | 0 |
| BL6xSD7 | Niere | **0** | 1. Messung | >50 | 29,59 | 0 | 0 |
| | | | 2. Messung | >50 | 28,91 | 0 | 0 |
| SD7xBL6 | Gehirn | **0** | 1. Messung | >50 | 28 | 0 | 0 |
| | | | 2. Messung | >50 | 27,59 | 0 | 0 |
| SD7xBL6 | Herz | **29** | 1. Messung | 28,17 | 27,82 | 44 | 38 |
| | | | 2. Messung | 28,5 | 27,38 | 32 | 20 |

(fortgesetzt)

| Kreuzungstyp | Gewebe | % Methylierung (Mittelwert) | | CT 640nm (CG-Sonde) | CT 705nm (tG-Sonde) | Ratio | % Methylierung (Einzelwerte) |
|---|---|---|---|---|---|---|---|
| SD7xBL6 | Darm | **42** | 1. Messung | 28,59 | 28,39 | 47 | 41 |
| | | | 2. Messung | 28,77 | 28,66 | 48 | 44 |
| SD7xBL6 | Niere | **37** | 1. Messung | 28,93 | 27,87 | 32 | 21 |
| | | | 2. Messung | 27,88 | 28,11 | 54 | 52 |
| SD7xBL6 | Leber | **47** | 1. Messung | 27,71 | 28,36 | 61 | 62 |
| | | | 2. Messung | 28,8 | 28,24 | 40 | 33 |

**Tabelle 5:** Ergebnisse des SD7-QM Assay. Gemessene CT für verschiedene Gewebetypen aus unterschiedlichen Kreuzungstypen sowie die daraus ermittelten Methylierungsraten.

| Kreuzungstyp | Gewebe | % Methylierung (Mittelwert) | | CT 640nm (CGSonde) | CT 705nm (tG-Sonde) | Ratio | % Methylierung (Einzelwerte) |
|---|---|---|---|---|---|---|---|
| BL6xSD7 | Herz | **29** | 1. Messung | 29,33 | 28,71 | 39 | 36 |
| | | | 2. Messung | 29,83 | 28,62 | 30 | 22 |
| BL6xSD7 | Darm | **38** | 1. Messung | 28,83 | 28,13 | 38 | 34 |
| | | | 2. Messung | 28,42 | 28,04 | 43 | 42 |
| BL6xSD7 | Niere | **26** | 1. Messung | 28,82 | 28 | 36 | 31 |
| | | | 2. Messung | 29,19 | 27,91 | 29 | 21 |
| SD7xBL6 | Gehim | **0** | 1. Messung | >50 | 28,57 | 0 | 0 |
| | | | 2. Messung | >50 | 28,77 | 0 | 0 |
| SD7xBL6 | Herz | **0** | 1. Messung | >50 | 28,24 | 0 | 0 |
| | | | 2. Messung | >50 | 28,47 | 0 | 0 |
| SD7xBL6 | Darm | **0** | 1. Messung | >50 | 28,96 | 0 | 0 |
| | | | 2. Messung | >50 | 29,37 | 0 | 0 |

(fortgesetzt)

| Kreuzungstyp | Gewebe | % Methylierung (Mittelwert) | | CT 640nm (CGSonde) | CT 705nm (tG-Sonde) | Ratio | % Methylierung (Einzelwerte) |
|---|---|---|---|---|---|---|---|
| SD7xBL6 | Niere | **0** | 1. Messung | >50 | 29,4 | 0 | 0 |
| | | | 2. Messung | >50 | 29,86 | 0 | 0 |
| SD7xBL6 | Leber | **0** | 1. Messung | >50 | 29,17 | 0 | 0 |
| | | | 2. Messung | >50 | 29,74 | 0 | 0 |

**Beschreibung der Abbildungen**

**Abbildung 1:**

Amplifikationskurve der beschriebenen Real Time PCR im Kanal 530 nm. Die Detektion der Gesamt-DNA erfolgt unabhängig vom Methylierungsstatus durch Verwendung einer methylierungsunspezifischen TaqMan-Sonde (SeqID-3). 1ng methylierte DNA (gekreuzte Linien) wurden mit CT 36,0 und 36,5 gemessen. Die Signale für 100ng unmethylierte DNA (gepunktete Linien) wurden bei CT 26,5 und 27,5 detektiert.

**Abbildung 2:**

Amplifikationskurve der beschriebenen Real Time PCR im Kanal 640 nm. Die methylierungsspezifische Light-Cycler Sonde (seqID-4) dient dem Nachweis der methylierten DNA. 1ng methylierte DNA (gekreuzte Linien) wurden mit einem CT von 36,0 und 36,5 gemessen. Keine Signale wurden in den Reaktionen mit 100ng unmethylierter DNA (gepunktete Linien) gemessen.

**Abbildung 3:**

Schematische Darstellung eines allelspezifischen quantitativen Methylierungsassay (Allelspezifischer Qm Assay). Sonde A bindet an das Amplifikat, wenn das Allel A enthalten (Teil A und B). Sonde B bindet benachbart zu Sonde A, wenn die Sequenz methylierte CpG Positionen enthielt (Teil A). Durch Energie-Resonanz-Energie-Transfer (FRET) wird ein Signal bei Wellenlänge 640 nm erzeugt. War die Ursprungssequenz nicht methyliert, bindet Sonde C (Teil B) und es wird ein Signal bei 705 nm erzeugt. Ist das Allel A nicht im Amplifikat enthalten, so wird Sonde A nicht binden und kein Signal erzeugt (Teil C und D).

**Abbildung 4:**

Kalibrierung des BL6 spezifischen QM Assay. Methylierte DNA wird im Kanal Ch2 bei 640 (Abb. 4a) unmethylierte DNA Kanal Ch3 (705 nm) (Abb. 4b) detektiert. Amplifiziert wurden 10 ng bisulfit-konvertierte DNA von Mischungen mit 0, 5, 10, 25, 50, 75 95 und 100 % Methylierung. Es wurde die Differenz der CT der Kanäle Ch2 und Ch3 für jeden Meßpunkt ermittelt und in die Formel Ratio=100/(1+2exp(deltaCT)) eingesetzt. Der erhaltene Meßwert (Ratio) wurde gegen die prozentuale Methylierung der DNA Mischungen aufgetragen (Abb. 4c). Steigung und Achsenabschnitt der Ausgleichgerade wurden anschließend für die Berechnung der Methylierungsraten von Gewebeproben benutzt.

**Abbildung 5:**

Kalibrierung für des SD7 spezifischen QM Assay. Kalibrierung des BL6 spezifischen QM Assay. Methylierte DNA wird im Kanal Ch2 bei 640 (Abb. 5a), unmethylierte DNA Kanal Ch3 (705 nm) (Abb. 5b) detektiert. Amplifiziert wurden 10 ng bisulfit-konvertierte DNA von Mischungen mit 0, 5, 10, 25, 50, 75 95 und 100 % Methylierung. Es wurde die Differenz der CT der Kanäle Ch2 und Ch3 für jeden Meßpunkt ermittelt und in die Formel Ratio=100/(1+2exp (deltaCT)) eingesetzt. Der erhaltene Meßwert (Ratio) wurde gegen die prozentuale Methylierung der DNA Mischungen aufgetragen (Abb. 5c). Steigung und Achsenabschnitt der Ausgleichgerade wurden anschließend für die Berechnung der Methylierungsraten von Gewebeproben benutzt.

**Abbildung 6:**

Übersicht über die allelspezifischen Methylierungsraten aus verschiedenen Gewebeproben. Teil A der Abbildung zeigt die Ergebnisse des BL6 spezifischen QM Assay. In Teil B sind die Ergebnisse des SD7 spezifischen QM Assay dargestellt. Die Methylierung auf den maternalen (mütterlich) vererbten Chromosomen wurde in beiden Kreuzungsvarainten mit 0% gemessen. Für das parental (väterlich) vererbte Chromosom wurden vom Gewebetyp abhängige Methylierungsraten zwischen 0% und 47% gefunden.

## Patentansprüche

1. Verfahren zur Quantifizierung von Cytosinmethylierungen, **dadurch gekennzeichnet, dass** folgende Schritte durchgeführt werden:

   a) die zu untersuchende DNA wird so umgesetzt, dass 5-Methylcytosin unverändert bleibt, während unmethyliertes Cytosin in Uracil oder in eine andere Base umgewandelt wird, die sich im Basenpaarungsverhalten von Cytosin unterscheidet,
   b) die umgewandelte DNA wird in Gegenwart zweier Real-Time-Sonden amplifiziert, wobei

      (i) die eine Sonde spezifisch für entweder den methylierten oder den nichtmethylierten Zustand der untersuchten DNA ist,
      (ii) die andere Sonde methylierungsunspezifisch ist,
      (iii) eine der beiden Sonden einen Donorfarbstoff trägt, der den Farbstoff der anderen Sonde über einen FRET anregt, sobald die beiden Sonden an das Amplifikat hybridisieren, und
      (iv) die Sonde, die den Donorfarbstoff trägt, eine Taqman-Sonde ist, bei der der Farbstoff am 3'Ende und der Quencher in 5'Richtung von dem Farbstoff lokalisiert ist,

   c) zu unterschiedlichen Zeitpunkten wird mittels einer Detektion der hybridisierten Sonden festgestellt, wieweit die Amplifikation fortgeschritten ist,
   d) mit Hilfe der Signale wird der Methylierungsgrad der untersuchten DNA bestimmt.

2. Verfahren nach Anspruch 1, **dadurch gekennzeichnet, dass** als methylierungsunspezifische Sonde die Taqman-Sonde benutzt wird.

3. Verfahren nach Anspruch 2, **dadurch gekennzeichnet, dass** als methylierungsspezifische Sonde eine Lightcycler-Sonde verwendet wird.

4. Verfahren nach einem der Ansprüche 1-3, **dadurch gekennzeichnet, dass** die Quantifizierung zur Diagnose von Krebserkrankungen oder anderen mit einer Veränderung des Methylierungsstatus assoziierten Krankheiten erfolgt.

5. Verfahren nach mindestens einem der Ansprüche 1-3, **dadurch gekennzeichnet, dass** die Quantifizierung zur Vorhersage von unerwünschten Arzneimittelwirkungen und zur Unterscheidung von Zelltypen oder Geweben, oder zur Untersuchung der Zelldifferenzierung erfolgt.

6. Ein Kit zur Durchführung eines der Verfahren nach den oben genannten Ansprüchen, der aus

   a) zwei Primern;
   b) einer Polymerase; und
   c) zwei Real-Time-Sonden besteht, wobei

      (i) die eine Sonde spezifisch für entweder den methylierten oder den nichtmethylierten Zustand der untersuchten DNA ist,
      (ii) die andere Sonde methylierungsunspezifisch ist,
      (iii) eine der beiden Sonden einen Donorfarbstoff trägt, der den Farbstoff der anderen Sonde, die eine Lightcycler-Sonde ist, über einen FRET anregt, sobald die beiden Sonden an das Amplifikat hybridisieren, und
      (iv) die Sonde, die den Donorfarbstoff trägt, eine Taqman-Sonde ist, bei der der Farbstoff am 3'Ende und der Quencher in 5'Richtung von dem Farbstoff lokalisiert ist;

   d) sowie optional weitere für eine PCR erforderliche Reagenzien und/oder ein Bisulfitreagenz enthält.

7. Kit nach Anspruch 6, **dadurch gekennzeichnet, dass** mindestens eine der Sonde isoliert von der anderen detektierbar ist.

## Claims

1. A method for the quantification of cytosine methylations, **characterized in that** the following steps are carried out:

    a) the DNA to be examined is transcribed in such a way that 5-methylcytosine remains unmodified whereas non-methylated cytosine is converted to uracil or another base which differs from cytosine with respect to its base pairing behaviour,
    b) the transcribed DNA is amplified in the presence of two real time probes, wherein

        (i) the one probe is specific to the methylated or the non-methylated state of the examined DNA,
        (ii) the other probe is methylation-unspecific,
        (iii) one of the two probes carries a donor dye which excites the dye of the other probe via a FRET approach as soon as the two probes hybridize to the amplificate, and
        (iv) the probe carrying the donor dye is a TaqMan probe where the dye is localized at the 3' end and the quencher in 5' direction from the dye,

    c) the progression of the amplification is determined at different points in time by means of a detection of the hybridized probes,
    d) the methylation degree of the DNA examined is determined with the assistance of the signals.

2. The method according to claim 1, **characterized in that** the TaqMan probe is used as a methylation-unspecific probe.

3. The method according to claim 2, **characterized in that** a LightCycler probe is used as a methylation-specific probe.

4. The method according to one of claims 1-3, **characterized in that** the quantification is carried out for the diagnosis of cancers or other diseases associated with a modification of the methylation state.

5. The method according to at least one of claims 1-3, **characterized in that** the quantification is carried out for the prediction of undesired effects of drugs, or for the differentiation of cell types or tissues, or for the examination of the cell differentiation.

6. A kit for the execution of one of the methods according to the above claims, consisting of:

    a) two primers;
    b) one polymerase; and
    c) two real time probes, wherein

        (i) the one probe is specific to the methylated or the non-methylated state of the examined DNA,
        (ii) the other probe is methylation-unspecific,
        (iii) one of the two probes carries a donor dye which excites the dye of the other probe, which is a Lightcycler probe, via a FRET approach as soon as the two probes hybridize to the amplificate, and
        (iv) the probe carrying the donor dye is a TaqMan probe where the dye is localized at the 3' end and the quencher in 5' direction from the dye,

    d) and optionally includes further reagents required for a PCR and/or a bisulfite reagent.

7. The kit according to claim 6, **characterized in that** at least one of the probes can be detected separately from the other probe.

## Revendications

1. Procédé de quantification de méthylations de cytosine, **caractérisé en ce que** les étapes suivantes sont réalisées :

    a) l'ADN à analyser est modifié de manière à ce que la 5-méthylcytosine reste inchangée, tandis que la cytosine non méthylée est transformée en uracile ou en une autre base qui se distingue de la cytosine en ce qui concerne son comportement en appariement de bases,
    b) l'ADN transformé est amplifié en présence de deux sondes en temps réel, étant donné que

(i) l'une des sondes est spécifique soit pour l'état méthylé soit pour l'état non méthylé de l'ADN analysé,
(ii) l'autre sonde est non spécifique à la méthylation,
(iii) une des deux sondes porte un colorant donneur qui excite le colorant de l'autre sonde via un FRET dès que les deux sondes s'hybrident sur l'amplificateur, et
(iv) la sonde qui porte le colorant donneur est une sonde TaqMan pour laquelle le colorant est localisé à l'extrémité 3' et l'agent d'étouffement est localisé dans le sens 5' du colorant,

c) on constate à des moments différents, par détection des sondes hybridées, jusqu'à quel niveau l'amplification a avancé,
d) le degré de méthylation de l'ADN analysée est déterminé à l'aide des signaux.

**2.** Procédé selon la revendication 1, **caractérisé en ce qu'**on utilise la sonde TaqMan comme sonde non spécifique à la méthylation.

**3.** Procédé selon la revendication 2, **caractérisé en ce qu'**on utilise une sonde Lightcycler comme sonde spécifique à la méthylation.

**4.** Procédé selon l'une quelconque des revendications 1 à 3, **caractérisé en ce que** la quantification est réalisée pour le diagnostic de cancers ou d'autres maladies associées à une modification de l'état de méthylation.

**5.** Procédé selon l'une quelconque des revendications 1 à 3, **caractérisé en ce que** la quantification est réalisée pour la prédiction d'effets indésirables de médicaments et pour la différenciation de types cellulaires ou de tissus, ou pour l'analyse de la différenciation cellulaire.

**6.** Kit pour la réalisation d'un des procédés selon les revendications ci-dessus, se composant de

a) deux amorces ;
b) une polymérase ; et
c) deux sondes en temps réel, étant donné que

(i) l'une des sondes est spécifique soit pour l'état méthylé soit pour l'état non méthylé de l'ADN analysé,
(ii) l'autre sonde est non spécifique à la méthylation,
(iii) une des deux sondes porte un colorant donneur qui excite le colorant de l'autre sonde, qui est une sonde Lightcycler, via un FRET dès que les deux sondes s'hybrident sur l'amplificateur, et
(iv) la sonde qui porte le colorant donneur est une sonde TaqMan pour laquelle le colorant est localisé à l'extrémité 3' et l'agent d'étouffement est localisé dans le sens 5' du colorant ;

d) ainsi que comprend en option d'autres réactifs nécessaires pour une PCR et/ou un réactif au bisulfite.

**7.** Kit selon la revendication 6, **caractérisé en ce que** au moins une des sondes est détectable séparément de l'autre.

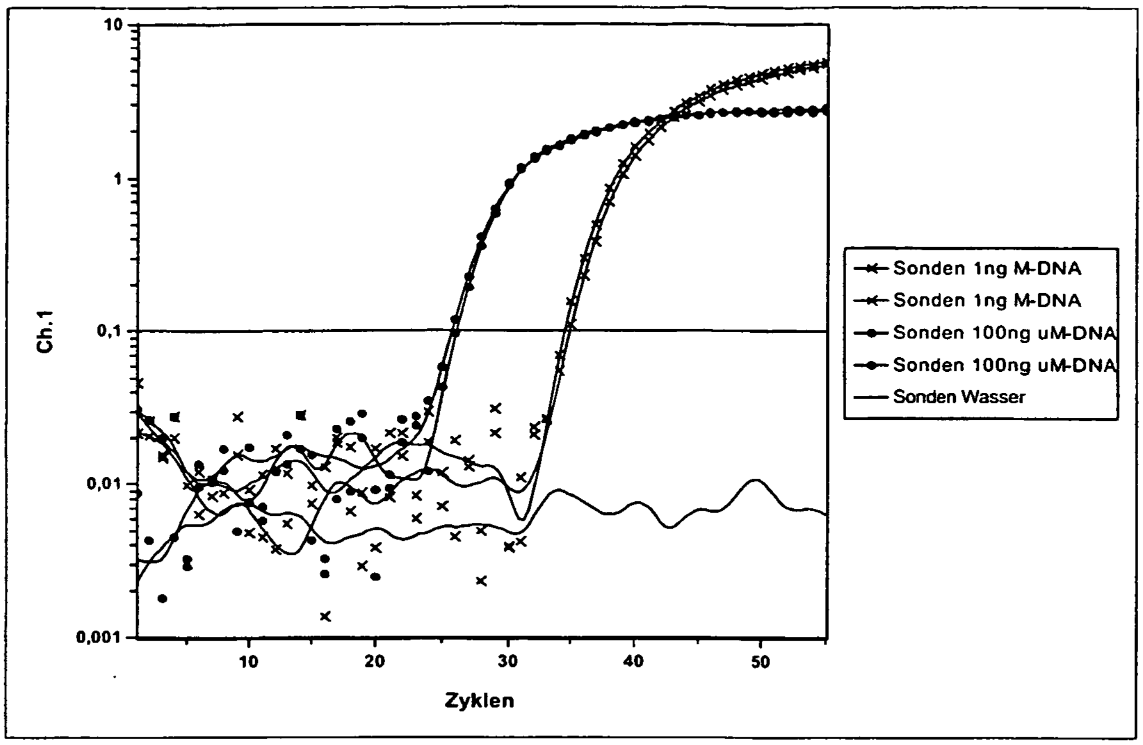

10
1
0,1
0,01
0,001
Ch.1
10
20
30
40
50
Zyklen
Sonden 1ng M-DNA
Sonden 1ng M-DNA
Sonden 100ng uM-DNA
Sonden 100ng uM-DNA
Sonden Wasser


Abb. 1

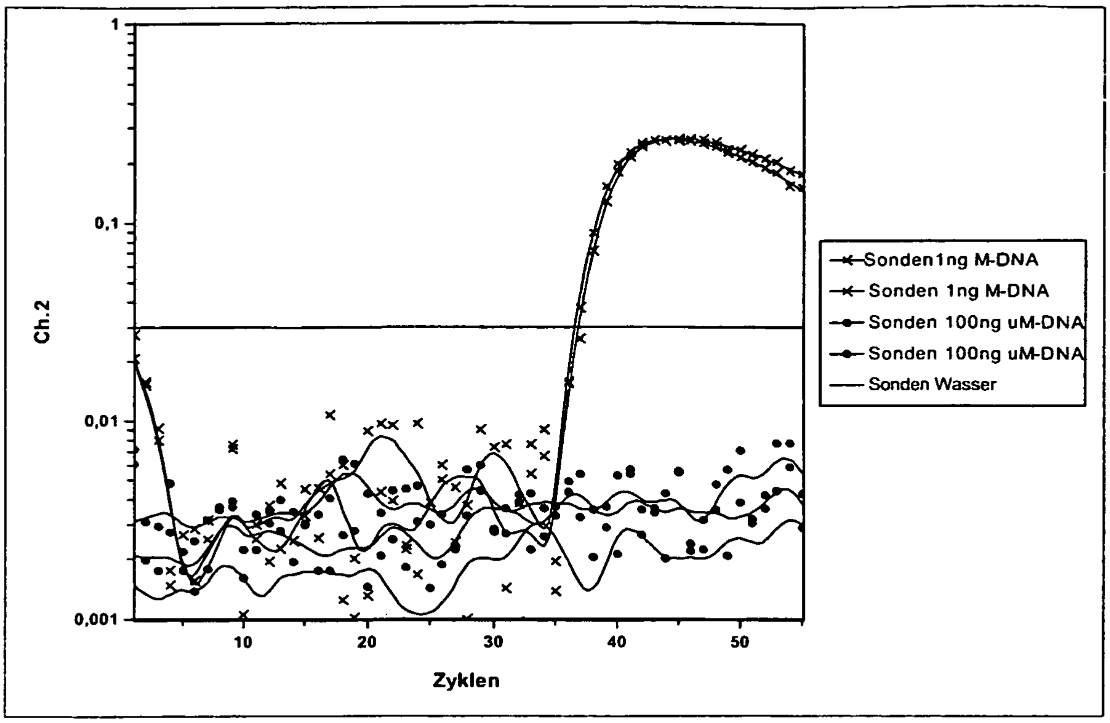

1
0,1
0,01
0,001
Ch.2
10
20
30
40
50
Zyklen
Sonden1ng M-DNA
Sonden 1ng M-DNA
Sonden 100ng uM-DNA
Sonden 100ng uM-DNA
Sonden Wasser


Abb. 2

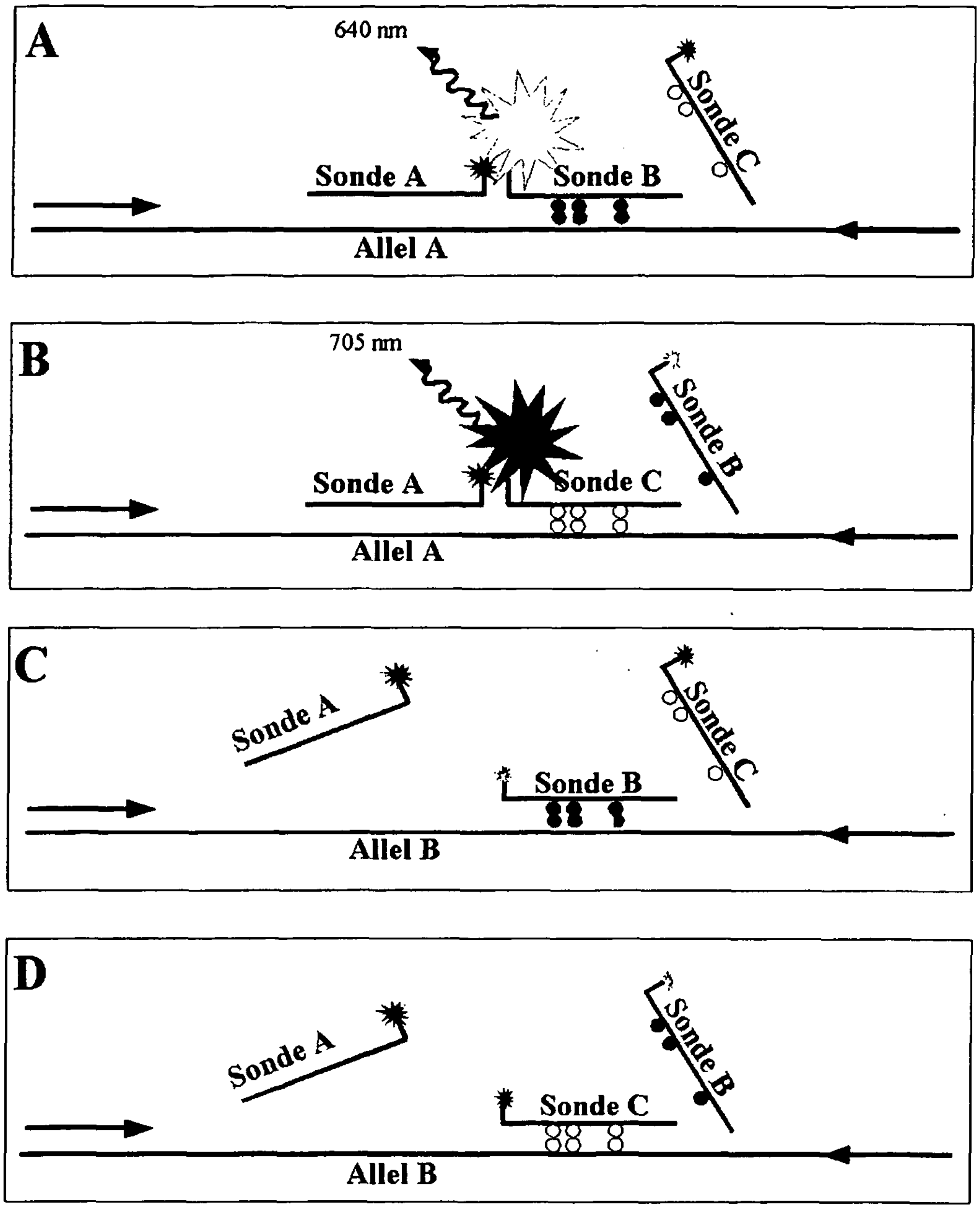
A
640 nm
Sonde A
Sonde B
Sonde C
Allel A
B
705 nm
Sonde A
Sonde C
Sonde B
Allel A
C
Sonde A
Sonde B
Sonde C
Allel B
D
Sonde A
Sonde C
Sonde B
Allel B

Abb. 3

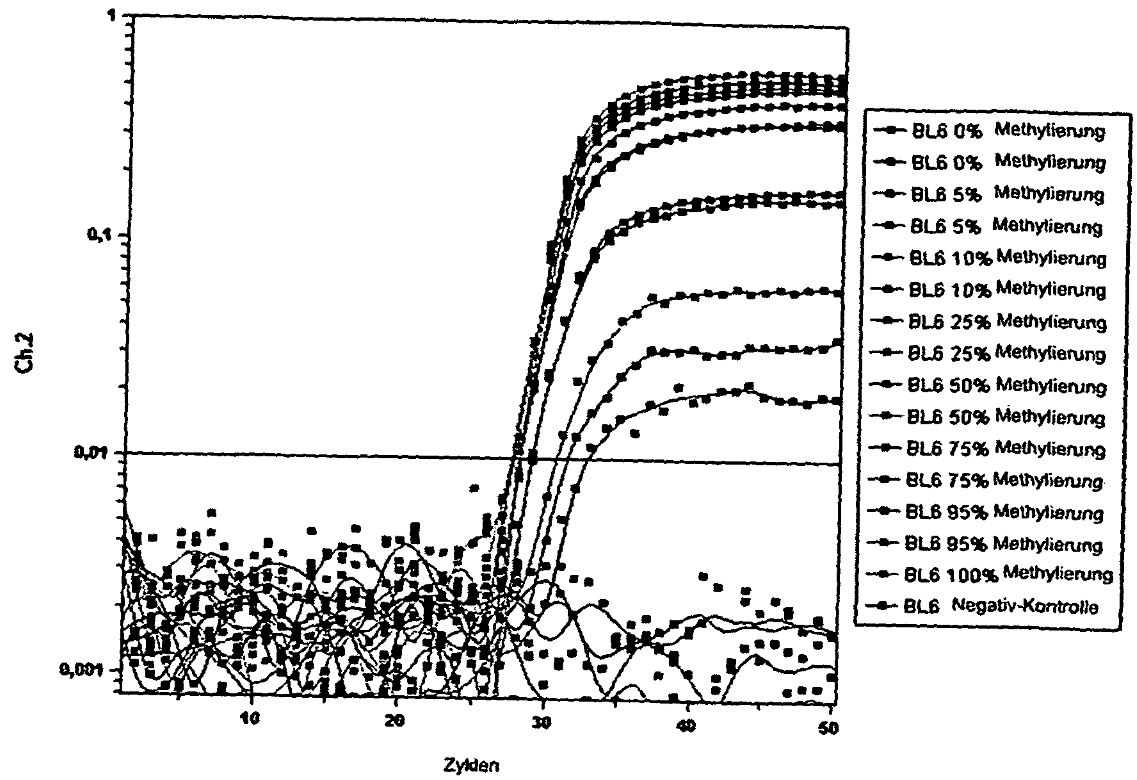
1
0,1
0,01
0,001
Ch.2
10
20
30
40
50
Zyklen
BL6 0% Methylierung
BL6 0% Methylierung
BL6 5% Methylierung
BL6 5% Methylierung
BL6 10% Methylierung
BL6 10% Methylierung
BL6 25% Methylierung
BL6 25% Methylierung
BL6 50% Methylierung
BL6 50% Methylierung
BL6 75% Methylierung
BL6 75% Methylierung
BL6 95% Methylierung
BL6 95% Methylierung
BL6 100% Methylierung
BL6 Negativ-Kontrolle

Abb. 4a

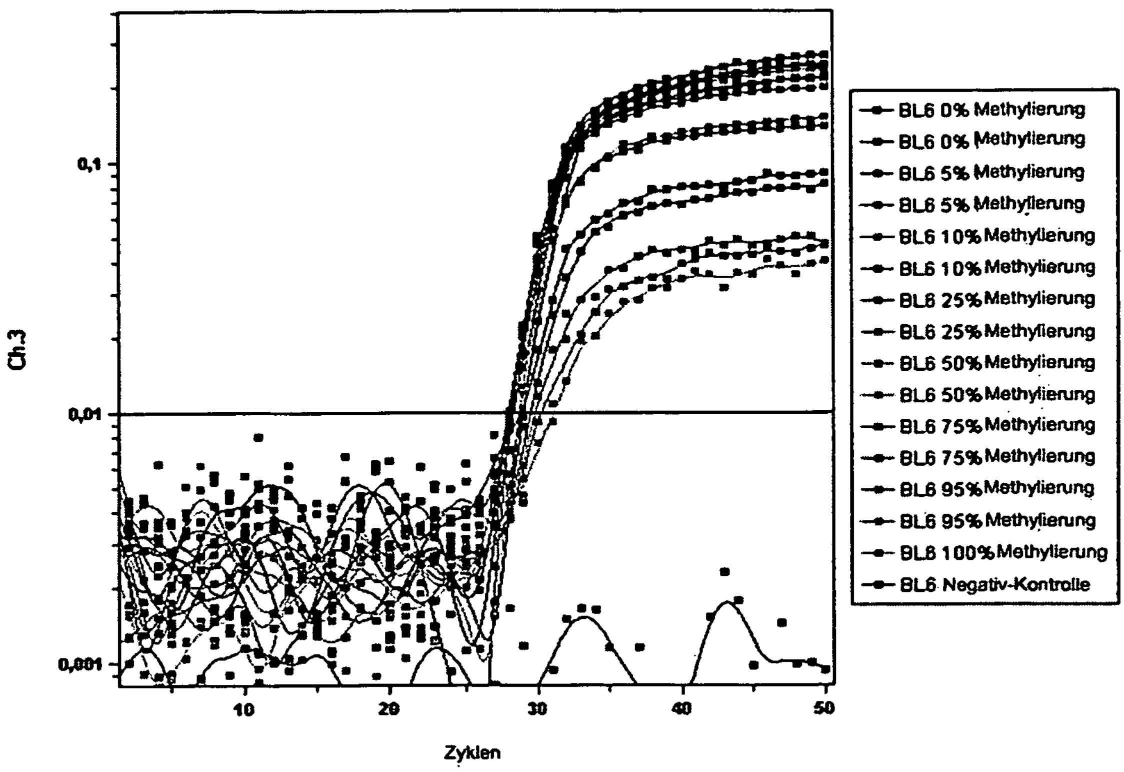
0,1
0,01
0,001
Ch.3
10
20
30
40
50
Zyklen
BL6 0% Methylierung
BL6 0% Methylierung
BL6 5% Methylierung
BL6 5% Methylierung
BL6 10% Methylierung
BL6 10% Methylierung
BL6 25% Methylierung
BL6 25% Methylierung
BL6 50% Methylierung
BL6 50% Methylierung
BL6 75% Methylierung
BL6 75% Methylierung
BL6 95% Methylierung
BL6 95% Methylierung
BL6 100% Methylierung
BL6 Negativ-Kontrolle

Abb. 4b

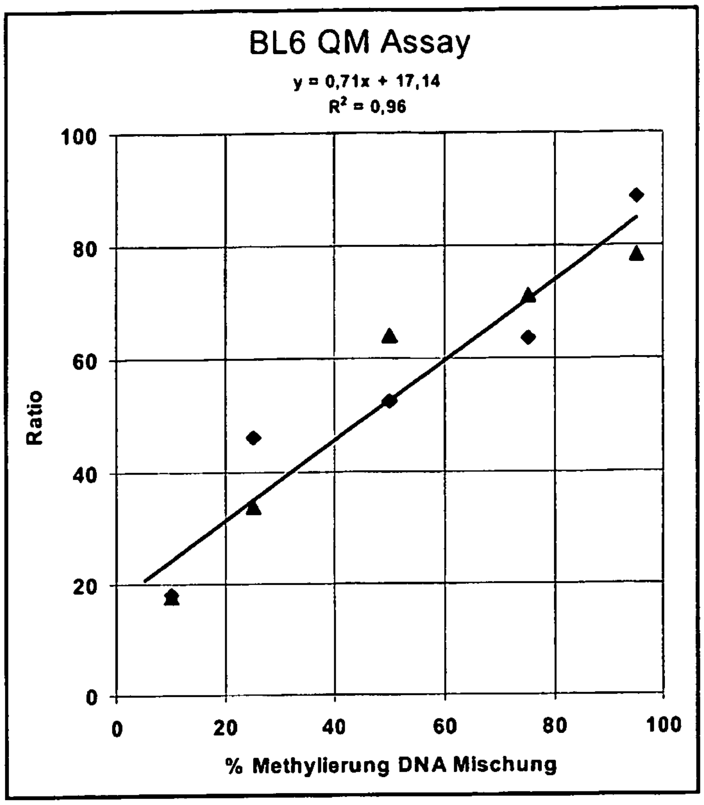
BL6 QM Assay
y = 0,71x + 17,14
R² = 0,96
100
80
60
40
20
0
Ratio
0
20
40
60
80
100
% Methylierung DNA Mischung

Abb. 4c

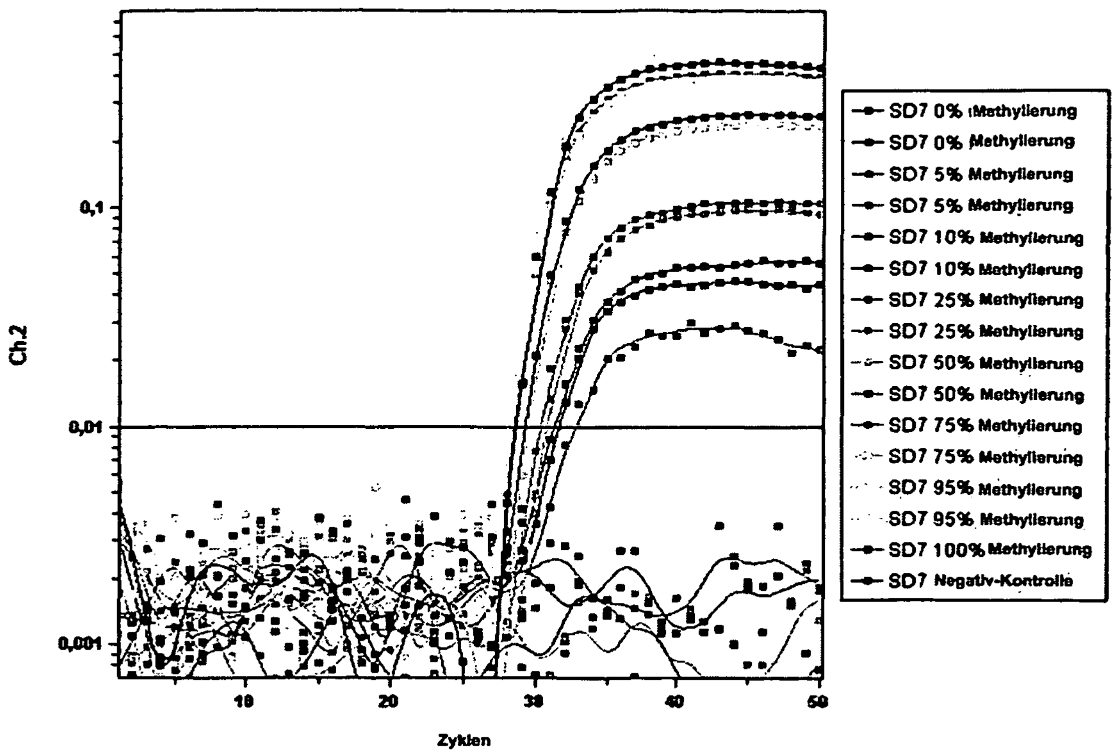
0,1
0,01
0,001
Ch.2
10
20
30
40
50
Zyklen
SD7 0% Methylierung
SD7 0% Methylierung
SD7 5% Methylierung
SD7 5% Methylierung
SD7 10% Methylierung
SD7 10% Methylierung
SD7 25% Methylierung
SD7 25% Methylierung
SD7 50% Methylierung
SD7 50% Methylierung
SD7 75% Methylierung
SD7 75% Methylierung
SD7 95% Methylierung
SD7 95% Methylierung
SD7 100% Methylierung
SD7 Negativ-Kontrolle

Abb. 5a

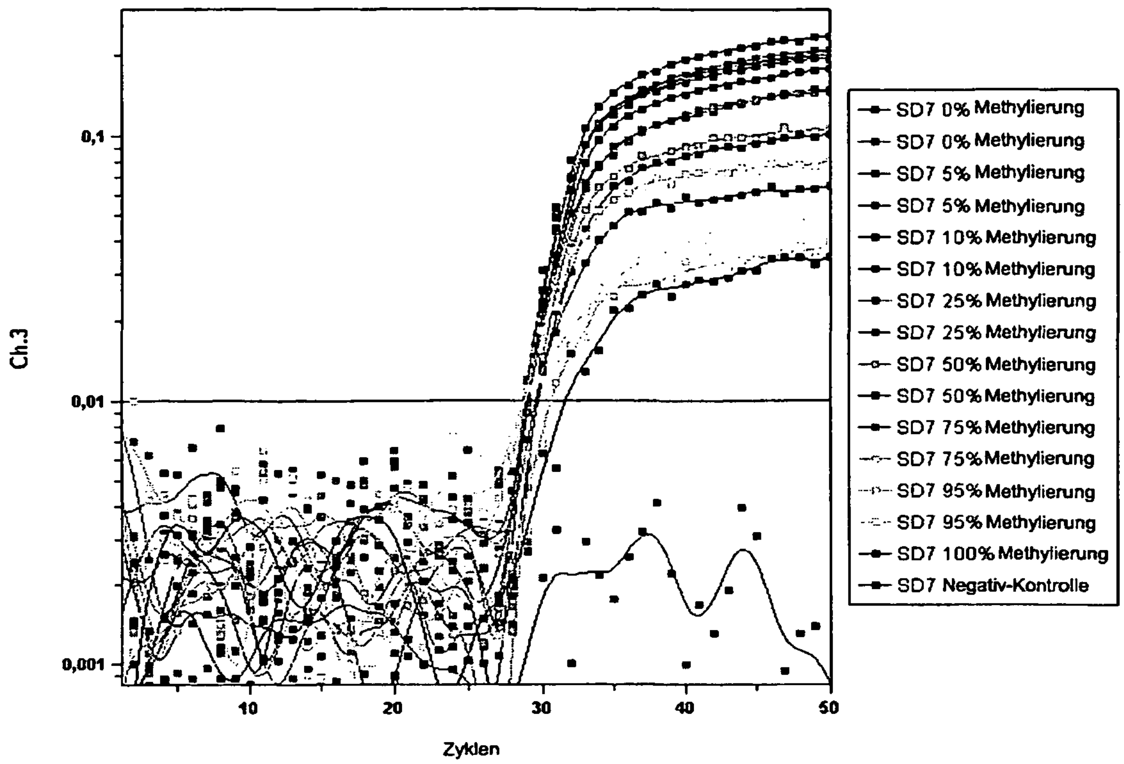
0,1
0,01
0,001
Ch.3
10
20
30
40
50
Zyklen
SD7 0% Methylierung
SD7 0% Methylierung
SD7 5% Methylierung
SD7 5% Methylierung
SD7 10% Methylierung
SD7 10% Methylierung
SD7 25% Methylierung
SD7 25% Methylierung
SD7 50% Methylierung
SD7 50% Methylierung
SD7 75% Methylierung
SD7 75% Methylierung
SD7 95% Methylierung
SD7 95% Methylierung
SD7 100% Methylierung
SD7 Negativ-Kontrolle

Abb. 5b

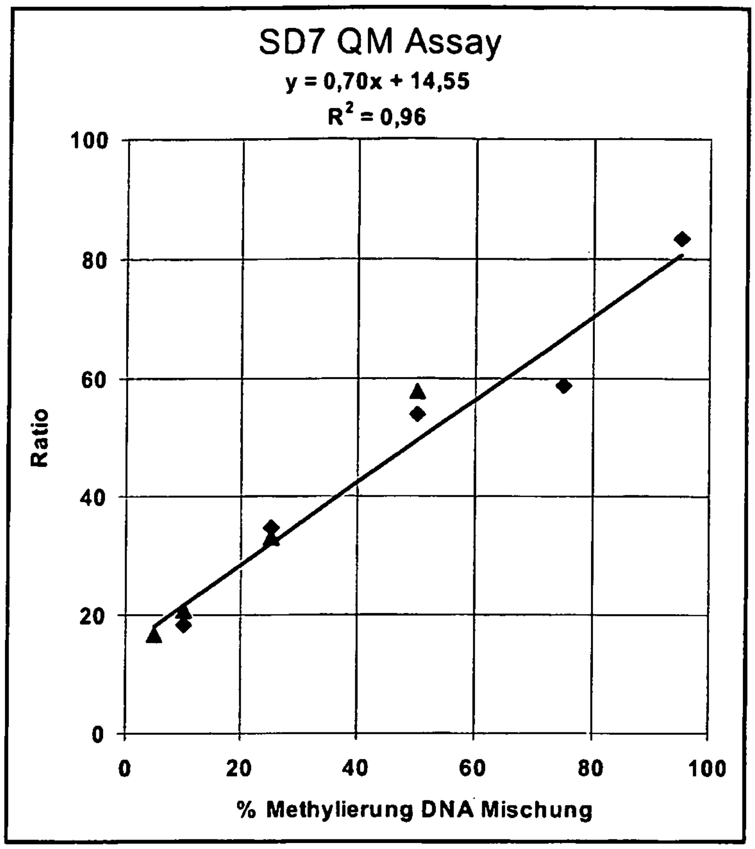
SD7 QM Assay
y = 0,70x + 14,55
$R^2$ = 0,96
100
80
60
40
20
0
Ratio
0
20
40
60
80
100
% Methylierung DNA Mischung

Abb. 5c

A

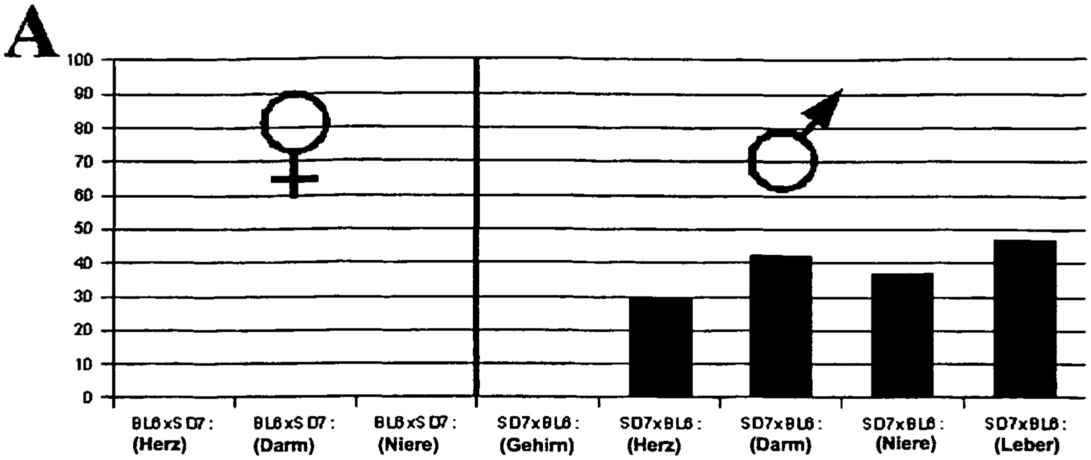
100
90
80
70
60
50
40
30
20
10
0
BL6xSD7: (Herz)
BL6xSD7: (Darm)
BL6xSD7: (Niere)
SD7xBL6: (Gehirn)
SD7xBL6: (Herz)
SD7xBL6: (Darm)
SD7xBL6: (Niere)
SD7xBL6: (Leber)

B

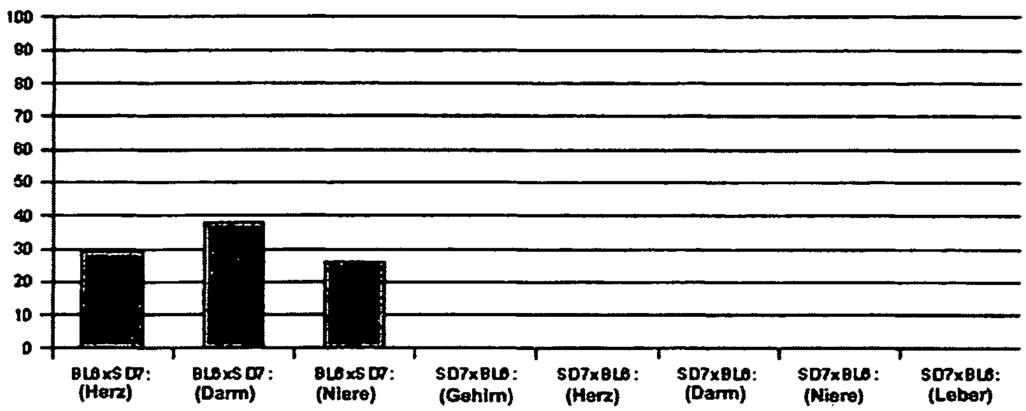
100
90
80
70
60
50
40
30
20
10
0
BL6xSD7: (Herz)
BL6xSD7: (Darm)
BL6xSD7: (Niere)
SD7xBL6: (Gehirn)
SD7xBL6: (Herz)
SD7xBL6: (Darm)
SD7xBL6: (Niere)
SD7xBL6: (Leber)

Abb. 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10154317 A1
- DE 10029915 A1
- WO 02072880 A
- WO 0070090 A
- US 6331393 B
- DE 10029915
- WO 2005038051 A
- US 6124120 A
- WO 200503805 A


### In der Beschreibung aufgeführte Nicht-Patentliteratur


- Five not four: History and significance of the fifth base. **Millar et al.** The Epigenome. Wiley-VCH Verlag, 2003, 3-20
- **Fraga ; Estella.** DNA methylation: a profile of methods and applications. *Biotechniques,* September 2002, vol. 33 (3), 632, 634, 636-49
- **Herman et al.** Methylation-specific PCR: a novel PCR assay for methylation status of CpG islands. *Proc Natl Acad Sei U S A.,* 03. September 1996, vol. 93 (18), 9821-6
- **Cottrell et al.** A real-time PCR assay for DNAmethylation using methylation-specific blockers. *Nucl. Acids. Res.,* 2004, vol. 32, e10
- **Trinh et al.** DNA methylation analysis by MethyLight technology. *Methods,* Dezember 2001, vol. 25 (4), 456-62
- **Kains.** The PCR plateau phase - towards an understanding of its limitations. *Biochem. Biophys. Acta,* 2000, vol. 1494, 23-27
- **Lehmann et al.** Quantitative assessment of promoter hypermethylation during breast cancer development. *Am J Pathol.,* Februar 2002, vol. 160 (2), 605-12
- **Eads et al.** *CANCER RESEARCH,* 15. April 2001, vol. 61, 3410-3418
- **Lehmann ; Kreipe.** Real-time PCR-based assay for quantitative determination of methylationstatus. *Methods Mol Biol,* 2004, vol. 287, 207-18
- **Zeschnigk et al.** A novel real-time PCR assay for quantitative analysis of methylated alleles (QAMA): analysis of the retinoblastoma locus. *Nucleic Acids Res.,* 07. September 2004, vol. 32 (16), e125
- **Frommer et al.** A genomic sequencing protocol that yields a positive display of 5-methylcytosine residues in individual DNA strands. *Proc Natl Acad Sei U S A.,* 01. Marz 1992, vol. 89 (5), 1827-31
- **Olek.** A modified and improved method for bisulphite based cytosine methylation analysis. *Nucleic Acids Res.,* 15. Dezember 1996, vol. 24 (24), 5064-6
- **Bransteitter et al.** Activation-induced cytidine deaminase deaminates deoxycytidine on singlestranded DNA but requires the action of RNase. *Proc Natl Acad Sci U S A.,* 01. April 2003, vol. 100 (7), 4102-7
- **Press, W. H. ; Teukolsky, S. A. ; Vetterling, W. T. ; Flannery, B. P.** Numerical Recipes in C. Cambridge. University Press, 2002
- **Hawkins et al.** Whole genome amplification--applications and advances. *Curr. Opin. Biotechnol.,* Februar 2002, vol. 13 (1), 65-7